# EUROPEAN PATENT SPECIFICATION

(11) **EP 4 206 104 B1**
(45) Date of publication and mention of the grant of the patent: **20.11.2024**
(21) Application number: 22213786.1
(22) Date of filing: 15.12.2022
(51) Int. Cl.: B65H 63/06, G01N 21/89, G01N 33/36, D01H 13/22

(54) **YARN MONITORING DEVICE**
GARNÜBERWACHUNGSVORRICHTUNG
DISPOSITIF DE SURVEILLANCE DE FIL

(30) Priority: 28.12.2021 JP 2021214717
(43) Date of publication of application: 05.07.2023
(73) Proprietor: Murata Machinery, Ltd., Kyoto-shi, Kyoto 601-8326 (JP)
(72) Inventor: MATSUMOTO, Keigo, Kyoto-shi, Kyoto, 612-8686 (JP); MIYAKE, Yasuo, Kyoto-shi, Kyoto, 612-8686 (JP); TSUKAMOTO, Shinichi, Kyoto-shi, Kyoto, 612-8686 (JP); NAKATANI, Masatoshi, Kyoto-shi, Kyoto, 612-8686 (JP)
(74) Representative: Hoffmann Eitle

(56) References cited:
- EP-A1- 3 130 553
- JP-A- 2013 253 342
- JP-A- 2017 036 140
- US-A1- 2003 070 481

## Description

### BACKGROUND OF THE INVENTION

### 1. Field of the Invention

The present invention relates to a yarn monitoring device.

### 2. Description of the Related Art

Conventionally, a technology related to a yarn monitoring device for measuring a state of a traveling yarn is known in the art. A yarn monitoring device disclosed in JP 2017-36140 A includes a holder in which a measurement sensor component is mounted to measure the state of the traveling yarn. A traveling area, which is an area through which a yarn travels, and a yarn path guide that causes the yarn to travel through the traveling area are provided in the holder. The yarn path guide guides the traveling yarn such that the traveling yarn passes through the traveling area. The yarn path guide is formed by using, for example, a wear-resistant material, such as ceramic or titanium, and has a high electrical resistivity.

### Problem to be solved by the Invention

In a yarn monitoring device disclosed in JP 2017-36140 A, when the yarn travels inside the yarn monitoring device, the yarn contacts the yarn path guide that is formed by using a material having a high electrical resistivity. Accordingly, static electricity is generated on the yarn path guide and the yarn. Such static electricity may affect the yarn monitoring device. For example, the static electricity may cause malfunction or damage to the yarn monitoring device. JP 2013 253342 A is related to a yarn path guide having a charge eliminating member.

### SUMMARY OF THE INVENTION

An object of the present invention is to provide a yarn monitoring device that suppresses an effect of static electricity.

According to one aspect of the present invention, a yarn monitoring device that monitors a state of a yarn that travels in a traveling direction through a traveling space includes the features of claim 1.

The above and other objects, features, advantages and technical and industrial significance of this invention will be better understood by reading the following detailed description of presently preferred embodiments of the invention, when considered in connection with the accompanying drawings.

### BRIEF DESCRIPTION OF THE DRAWINGS

FIG. 1 is a side view of a spinning machine according to a first embodiment of the present invention.
FIG. 2 is a perspective view of a yarn monitoring device for use in the spinning machine shown in FIG. 1.
FIG. 3 is a disassembled perspective view of the yarn monitoring device shown in FIG. 2.
FIG. 4 is a cross-sectional view of a yarn path guide of the yarn monitoring device shown in FIG. 3.
FIG. 5 is a disassembled perspective view of a yarn monitoring device according to a second embodiment of the present invention.
FIG. 6 is a disassembled perspective view of the yarn monitoring device shown in FIG. 5 from a different angle.
FIG. 7 is a plan view of the yarn monitoring device shown in FIG. 5.
FIG. 8 is a diagram showing a yarn path guide according to a modification.
FIG. 9 is a diagram showing a yarn path guide according to another modification.
FIGS. 10A, 10B, and 10C are cross-sectional views of a yarn path guide according to respective modifications.

### DETAILED DESCRIPTION

Exemplary embodiments of the present invention are explained below with reference to the accompanying drawings. Note that, identical elements are indicated by the same reference symbols in the drawings and redundant explanation thereof is omitted.

### First Embodiment

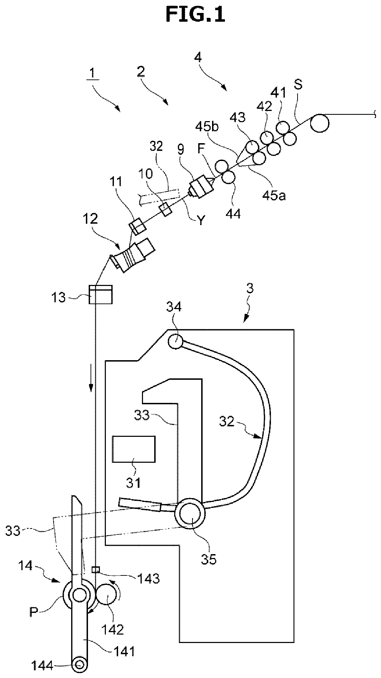

As shown in FIG. 1, the spinning machine 1 includes a spinning unit 2, a yarn joining cart 3, and a not-shown doffing cart. A plurality of the spinning units 2 shown in FIG. 1 are arranged side-by-side in a row. Each of the spinning units 2 generates the yarn Y and winds the yarn Y into a respective package P. If the yarn Y is cut or becomes discontinuous for some reason in a certain spinning unit 2, the yarn joining cart 3 performs a yarn joining operation in that spinning unit 2. When the package P becomes fully wound in a certain spinning unit 2, the doffing cart doffs the fully wound package P and supplies a new empty bobbin to that spinning unit 2.

As shown in FIG. 1, each of the spinning units 2 includes a drafting device 4, an air spinning device 9, a yarn monitoring device 10, a tension sensor 11, a yarn accumulating device 12, a waxing device 13, and a winding device 14 in this order from upstream in a traveling direction of the yarn Y. A not-shown unit controller is arranged for a predetermined number of the spinning units 2, and the unit controller controls operations of those spinning units 2.

The drafting device 4 drafts a sliver (a fiber bundle) S. The drafting device 4 includes a back roller pair 41, a third roller pair 42, a middle roller pair 43, and a front roller pair 44 in this order from upstream in a traveling direction of the sliver S. Each of the roller pairs 41, 42, 43, and 44 includes a bottom roller and a top roller. The bottom roller is rotationally driven by a driving motor arranged in each of the spinning units 2 and the like. An apron belt 45a is stretched over the bottom roller of the middle roller pair 43. An apron belt 45b is stretched over the top roller of the middle roller pair 43. In the following explanation, a side on which the apron belt 45b is provided with respect to the traveling yarn Y is referred to as a "front side" and an opposite side thereof is referred to as a "rear side".

The air spinning device 9 generates the yarn Y by applying twists by using a swirling air current to a fiber bundle F drafted by the drafting device 4. More particularly, although not shown, the air spinning device 9 includes a spinning chamber, a fiber guiding section, swirling air current generating nozzles, and a hollow guiding shaft. The fiber guiding section guides the fiber bundle F supplied from the drafting device 4, which is arranged upstream, to the inside of the spinning chamber. The swirling air current generating nozzles are arranged surrounding a path on which the fiber bundle F travels and produce a swirling air current inside the spinning chamber. The fiber ends of the fibers constituting the fiber bundle F are reversed and whirled by the swirling air current. The hollow guiding shaft guides the yarn Y from the inside of the spinning chamber to the outside of the air spinning device 9.

The yarn monitoring device 10 monitors, at a location between the air spinning device 9 and the yarn accumulating device 12, state of the traveling yarn Y, and detects presence / absence of a yarn defect in the yarn Y based on the monitored information about the state. Upon detecting the yarn defect, the yarn monitoring device 10 transmits a yarn-defect detection signal to the unit controller. The yarn monitoring device 10 detects, for example, a thickness abnormality of the yarn Y and / or a foreign substance contained in the yarn Y as the yarn defect. The yarn monitoring device 10 also detects a yarn breakage and the like. The tension sensor 11 measures, at a location between the air spinning device 9 and the yarn accumulating device 12, a tension of the traveling yarn Y, and transmits a tension measurement signal representing the measured tension to the unit controller. When the unit controller determines, based on a detection result obtained in the yarn monitoring device 10 and / or the tension sensor 11, that an abnormality is present in the yarn Y, the yarn Y is cut in that spinning unit 2. Specifically, the yarn Y is cut by suspending the generation of the yarn Y by stopping the supply of the air to the air spinning device 9. Alternatively, the yarn Y can be cut by using a dedicated cutter.

The waxing device 13 applies wax to the yarn Y at a location between the yarn accumulating device 12 and the winding device 14. The yarn accumulating device 12 accumulates the yarn Y at a location between the air spinning device 9 and the winding device 14.

The winding device 14 winds the yarn Y around a bobbin thereby forming the package P. The winding device 14 includes a cradle arm 141, a winding drum 142, and a traversing guide 143. The cradle arm 141 rotatably supports the bobbin. The cradle arm 141 is pivotably supported by a support shaft 144. By pivoting the cradle arm 141, a surface of the bobbin or a surface of the package P can be caused to contact a surface of the winding drum 142 at an appropriate pressure. A predetermined number of the winding drums 142 of the spinning unit 2 are driven all together by a not-shown driving motor. Accordingly, the bobbins or the packages P of a plurality of the spinning units 2 are rotated in a winding direction. The traversing guide 143 in each of a predetermined number of the spinning units 2 are arranged on a not-shown shaft 25 that is shared by those spinning units 2. The traversing guide 143 traverses the yarn Y by a predetermined width on the rotating bobbin or the package P when the driving motor causes the shaft 25 to perform a reciprocating movement along a direction of a rotation axis of the winding drum 142.

The yarn joining cart 3 travels to a certain spinning unit 2 in which the yarn Y is cut or becomes discontinuous for some reason and performs a yarn joining operation. The yarn joining cart 3 includes a yarn joining device 31, a suction pipe 32, and a suction mouth 33. The suction pipe 32 is pivotably supported by a support shaft 34. The suction pipe 32 catches the yarn Y from the air spinning device 9 and guides the caught yarn Y to the yarn joining device 31. The suction mouth 33 is pivotably supported by a support shaft 35. The suction mouth 33 catches the yarn Y from the winding device 14 and guides the caught yarn Y to the yarn joining device 31. The yarn joining device 31 joins the yarns Y guided thereto. The yarn joining device 31 can be a splicer that uses compressed air, a piecer that uses a seed yarn, a knotter that mechanically joins the yarn Y, and the like.

While the yarn joining cart 3 is performing the yarn joining operation, the package P is rotated in a direction that allows unwinding of the yarn Y from the package P (reverse rotation). When doing so, the cradle arm 141 is moved by a not-shown air cylinder so that the package P separates from the winding drum 142, and the package P is caused to perform the reverse rotation by a not-shown reverse-rotation roller arranged in the yarn joining cart 3.

The yarn monitoring device 10 according to the first embodiment is explained below in greater detail.

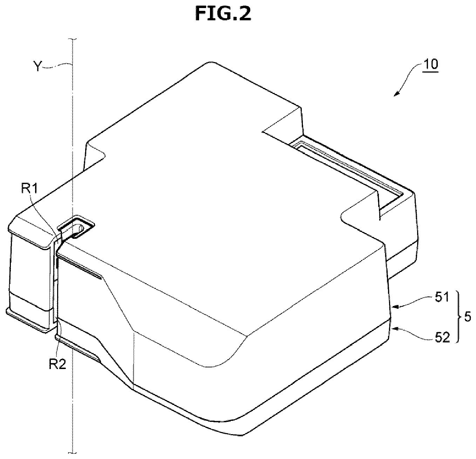

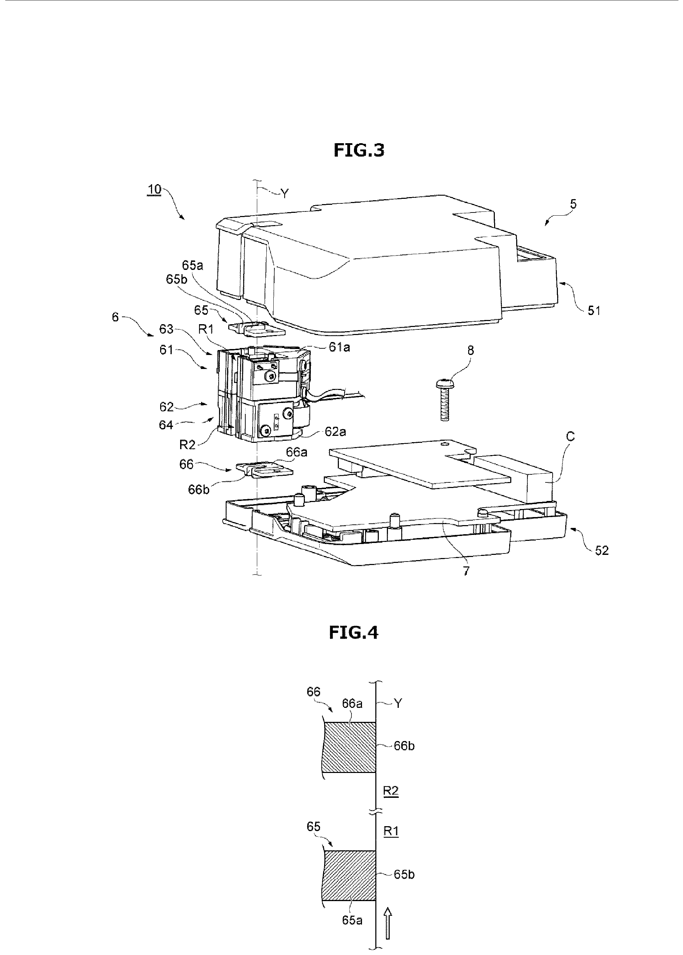

FIG. 2 is a perspective view of the yarn monitoring device 10 for use in the spinning machine 1. FIG. 3 is a disassembled perspective view of the yarn monitoring device 10. FIG. 4 is a schematic cross-sectional view of a later-explained upper yarn path guide 65 and a later-explained lower yarn path guide 66 provided in the yarn monitoring device 10. As shown in FIGS. 2 and 3, the yarn monitoring device 10 includes a housing 5, a yarn monitoring unit 6, a substrate 7, and a screw (first electrically conductive member) 8. The yarn monitoring unit 6, the substrate 7, and the screw 8 are arranged inside the housing 5. In the present specification, the "screw" can be rephrased as a bolt or a vis and the like. Fastening members such as screws, bolts, or vis are auxiliary materials required for the construction of the yarn monitoring device 10.

The housing 5 can be divided in an up-down direction. The housing 5 includes an upper housing (connecting member, upstream connecting member) 51 that is positioned on an upper side and a lower housing (connecting member, downstream connecting member) 52 that is positioned on a lower side. The housing 5 is formed, for example, by an electrically conductive resin. The upper housing 51 contacts the lower housing 52 when the housing 5 is in an assembled state (state shown in FIG. 2). A lower end edge of a side wall portion of the upper housing 51 and an upper end edge of a side wall portion of the lower housing 52 engage with each other and contact each other. The upper housing 51 and the lower housing 52 can be electrically connected to each other via an electrically conductive member other than the screw 8. The upper housing 51 and the lower housing 52 can be electrically connected to each other via a screw. An electrical resistivity of the upper housing 51 is higher than or equal to, for example, the electrical resistivity of the screw 8. The electrical resistivity of the lower housing 52 is higher than or equal to, for example, the electrical resistivity of the screw 8. However, in the present specification, "electrical conductivity" can be used as an indicator, instead of the electrical resistivity. The electrical conductivity is a reciprocal of the electrical resistivity. Furthermore, the term "electrically connected" means that a later-explained electric charge generated by the traveling of the yarn Y can be transferred.

The yarn monitoring unit 6 includes a first holder (holder) 61, a second holder (holder) 62, a first monitoring section (monitoring section) 63, a second monitoring section (monitoring section) 64, an upper yarn path guide (yarn path guide, upstream yarn path guide) 65, and a lower yarn path guide (yarn path guide, downstream yarn path guide) 66. Each of these components will be explained below.

The first holder 61 is arranged at an upstream side of the second holder 62. The first holder 61, for example, includes a main body 61a that is made of resin. A traveling space R1 through which the yarn Y travels is formed in the main body 61a of the first holder 61. The traveling space R1 is a space that extends along a traveling path of the yarn Y. The traveling space R1 opens on a front side. In other words, the traveling space R1 is a space through which the traveling yarn Y passes, extends from upstream to downstream, and is a groove that is open on the front side. When seen from the traveling direction of the yarn Y, the traveling space R1 is formed in a shape of English letter U.

The first monitoring section 63 is provided in the first holder 61. The first monitoring section 63 monitors a state of the yarn Y that is traveling through the traveling space R1. The first monitoring section 63 includes a foreign substance detection function that detects a foreign substance mixed into the traveling yarn Y. The first monitoring section 63 includes, for example, two not-shown light emitting elements and two not-shown light receiving elements. The two light emitting elements and the two light receiving elements are mounted inside the main body 61a, and are supported by the main body 61a. When seen from the traveling direction of the yarn Y, the two light emitting elements and the two light receiving elements are arranged at different positions around the traveling space R1. The two light emitting elements and the two light receiving elements are arranged such that optical axes thereof are positioned on the same plane that is orthogonal to the traveling direction of the yarn Y. The first monitoring section 63 monitors the state of the yarn Y by projecting light from each light emitting element onto the yarn Y that is traveling through the traveling space R1, and receiving the light reflected and / or transmitted from the yarn Y at the two light receiving elements. A light emitting diode (LED) and the like, for example, can be used as the light emitting element. A photodiode and the like, for example, can be used as the light receiving element.

The second holder 62 is arranged at the downstream side of the first holder 61. The second holder 62 includes, for example, a main body 62a that is made of resin. A traveling space R2 through which the yarn Y travels is formed in the main body 62a of the second holder 62. The traveling space R2 is a space that extends along a traveling path of the yarn Y. The traveling space R2 is continuous with the traveling space R1. The traveling space R2 opens on the front side. In other words, the traveling space R2 is a space through which the traveling yarn Y passes, extends from upstream to downstream, and is a groove that is open on the front side. When seen from the traveling direction of the yarn Y, the traveling space R2 is formed in a shape of English letter U.

The second monitoring section 64 is provided in the second holder 62. The second monitoring section 64 monitors a state of the yarn Y that is traveling through the traveling space R2. The second monitoring section 64 has a yarn thickness detection function that detects a thickness of the traveling yarn Y. The second monitoring section 64 includes, for example, a not-shown light emitting element and a not-shown light receiving element. The light emitting element and the light receiving element are mounted inside the main body 62a, and are supported by the main body 62a. The light emitting element and the light receiving element are arranged across the traveling space R2 and facing each other. The second monitoring section 64 monitors the state of the yarn Y by projecting light from the light emitting element onto the yarn Y that is traveling through the traveling space R2, and receiving the light transmitted from the yarn Y at the light receiving element. An LED and the like, for example, can be used as the light emitting element. A photodiode and the like, for example, can be used as the light receiving element.

The upper yarn path guide 65 is provided in the first holder 61, at the upstream side of the first holder 61 with respect to the traveling direction of the yarn Y. In the present embodiment, the upper yarn path guide 65 is arranged between the first holder 61 and the upper housing 51. The upper yarn path guide 65 is, for example, one of the members that form an exterior shape on the upstream side of the yarn monitoring device 10. The upper yarn path guide 65 is, for example, a rectangular plate member in which a grooved part (a groove having a shape of English letter U) that extends in a thickness direction thereof (traveling direction of the yarn Y) and opens at the front side is formed. The upper yarn path guide 65 includes an upper charge eliminating member (charge eliminating member, upstream charge eliminating member) 65a having a grooved part. A side surface (equivalent to plate thickness) of the grooved part of the upper charge eliminating member 65a is a guide surface 65b that guides the yarn Y. In other words, the upper charge eliminating member 65a constitutes the guide surface 65b that guides the yarn Y in the traveling space R1. As in an example shown in FIG. 4, the guide surface 65b always contacts the yarn Y, and regulates a traveling path of the yarn Y. Accordingly, the guide surface 65b guides the traveling yarn Y to the traveling space R1. The traveling yarn Y is pulled backward and pressed against the guide surface 65b that is a bottom of the grooved part. In this manner, a position of the yarn y is fixed.

The upper charge eliminating member 65a is, for example, made of an electrically conductive material. The upper charge eliminating member 65a eliminates an electric charge generated by the traveling of the yarn Y (generated in the yarn Y). The entire upper yarn path guide 65 can be the upper charge eliminating member 65a, or only a portion of the upper yarn path guide 65 (the portion forming the guide surface 65b) can be the upper charge eliminating member 65a. The upper charge eliminating member 65a is positioned near an upper end (entrance) of the traveling space R1, and is arranged near the traveling space R1. In the present embodiment, the upper charge eliminating member 65a is a part of the upper yarn path guide 65, and is arranged at a position where it contacts the traveling yarn Y. The material from which the upper charge eliminating member 65a is made is determined according to the compatibility of that material with the material of the yarn Y and a traveling speed of the yarn Y. The upper charge eliminating member 65a, for example, can be made of metal, such as stainless steel or copper, or can be made of an electrically conductive resin or an electrically conductive ceramic. Furthermore, the upper charge eliminating member 65a can be arranged at a position where it does not contact the traveling yarn Y. In other words, the upper charge eliminating member 65a can be provided in a portion other than the guide surface 65b. In that case, it is sufficient that the upper charge eliminating member 65a is arranged near the yarn Y such that the static electricity can be eliminated from the yarn Y. For example, it is sufficient that the upper charge eliminating member 65a is arranged near the yarn Y with a small gap between itself and the yarn Y. The electrical resistivity of the upper charge eliminating member 65a is, for example, higher than or equal to the electrical resistivity of the upper housing 51. The electrical resistivity of the upper charge eliminating member 65a is, for example, 1.0 × 10⁶ Ohm-meters (Ω·m) or less.

The lower yarn path guide 66 is provided in the second holder 62, at the downstream side of the second holder 62 with respect to the traveling direction of the yarn Y. In the present embodiment, the lower yarn path guide 66 is arranged between the second holder 62 and the lower housing 52. The lower yarn path guide 66 is, for example, one of the members that form an exterior shape on the downstream side of the yarn monitoring device 10. The lower yarn path guide 66 is, for example, a rectangular plate member in which a grooved part (a groove having a shape of English letter U) that extends in a thickness direction thereof (traveling direction of the yarn Y) and opens at the front side is formed. The configuration of the lower yarn path guide 66 is the same as that of the upper yarn path guide 65, and includes a lower charge eliminating member (charge eliminating member, downstream charge eliminating member) 66a having a grooved part. A side surface (equivalent to plate thickness) of the grooved part of the lower charge eliminating member 66a is a guide surface 66b that guides the yarn Y. In other words, the lower charge eliminating member 66a constitutes the guide surface 66b that guides the yarn Y from the traveling space R2 to the outside of the yarn monitoring device 10. As in an example shown in FIG. 4, the guide surface 66b always contacts the yarn Y, and regulates a traveling path of the yarn Y. Accordingly, the guide surface 66b guides the traveling yarn Y from the traveling space R2 to the outside of the yarn monitoring device 10. The traveling yarn Y is pulled backward and pressed against the guide surface 66b that is a bottom of the grooved part. In this manner, the position of the yarn y is defined. The lower yarn path guide 66 is arranged such that the upstream side and downstream side of the lower yarn path guide 66 are inverted vertically with respect to the upper yarn path guide 65. In other words, the upper yarn path guide 65 and the lower yarn path guide 66 are arranged so as to face each other in a reversed manner in the up-down direction.

The lower charge eliminating member 66a is, for example, made of an electrically conductive material. The lower charge eliminating member 66a eliminates the electric charge generated by the traveling of the yarn Y. The entire lower yarn path guide 66 can be the lower charge eliminating member 66a, or only a portion of the lower yarn path guide 66 (the portion forming the guide surface 66b) can be the lower charge eliminating member 66a. The lower charge eliminating member 66a is positioned near a lower end (exit) of the traveling space R2, and is arranged near the traveling space R2. In the present embodiment, the lower charge eliminating member 66a is a part of the lower yarn path guide 66, and is arranged at a position where it contacts the traveling yarn Y. The material from which the lower charge eliminating member 66a is made is determined according to the compatibility of that material with the material of the yarn Y and a traveling speed of the yarn Y. The lower charge eliminating member 66a, for example, can be made of metal, such as stainless steel or copper, or can be made of an electrically conductive resin or an electrically conductive ceramic. Furthermore, the lower charge eliminating member 66a can be arranged at a position where it does not contact the traveling yarn Y. In other words, the lower charge eliminating member 66a can be provided in a portion other than the guide surface 66b. In that case, it is sufficient that the lower charge eliminating member 66a is arranged near the yarn Y such that the static electricity can be eliminated from the yarn Y. For example, it is sufficient that the upper charge eliminating member 65a is arranged near the yarn Y with a small gap between itself and the yarn Y. The electrical resistivity of the lower charge eliminating member 66a is, for example, higher than or equal to the electrical resistivity of the lower housing 52. The electrical resistivity of the lower charge eliminating member 66a is, for example, 1.0 × 10⁶ Ω·m or less.

The substrate 7 transmits information acquired by the first monitoring section 63 and the second monitoring section 64 to the outside of the yarn monitoring device 10. Inside the housing 5, the substrate 7 extends on a plane that is orthogonal to the traveling direction of the yarn Y. The substrate 7 contacts the screw 8 and a structure C.

The upper charge eliminating member 65a is, for example, electrically connected to a ground potential. In the present embodiment, the upper charge eliminating member 65a contacts the upper housing 51, and is electrically connected to the upper housing 51. The upper housing 51 is electrically connected to the ground potential. Specifically, the upper housing 51 that contacts the upper charge eliminating member 65a contacts the lower housing 52 and thus electrically connected to the lower housing 52. Moreover, the lower housing 52 is electrically connected to the substrate 7 via the screw 8 (see FIG. 3), and the substrate 7 is electrically connected to the ground potential via the structure C. In other words, the upper charge eliminating member 65a is electrically connected to the outside of the yarn monitoring device 10. Accordingly, the electric charge removed from the yarn Y by the upper charge eliminating member 65a is guided to the outside of the yarn monitoring device 10.

Furthermore, effect of external electromagnetic waves can be reduced by making the electrical potential of the substrate 7 as same as the electrical potential of the upper housing 51 and the lower housing 52. Moreover, because it is sufficient that the upper charge eliminating member 65a is electrically connected to the ground potential, the upper charge eliminating member 65a can be connected to the ground potential (earth) by using a path other than that explained above. In the present specification, the "ground potential" can be expressed as Frame Ground (FG), or simply as Ground (GND).

The lower charge eliminating member 66a is, for example, electrically connected to the ground potential. In the present embodiment, the lower charge eliminating member 66a contacts the lower housing 52, and is electrically connected to the lower housing 52. The lower housing 52 is electrically connected to the ground potential. Specifically, the lower housing 52 that contacts the lower charge eliminating member 66a is electrically connected to the substrate 7 via the screw 8. Moreover, the substrate 7 is electrically connected to the ground potential via the structure C. In other words, the lower charge eliminating member 66a is electrically connected to the outside of the yarn monitoring device 10. The electric charge removed from the yarn Y by the lower charge eliminating member 66a is guided to the outside of the yarn monitoring device 10. However, because it is sufficient that the lower charge eliminating member 66a is electrically connected to the ground potential, the lower charge eliminating member 66a can be connected to the ground potential by a path other than that explained above. The lower charge eliminating member 66a can be connected to the ground potential (earth) by a path other than that explained above. Moreover, because it is sufficient that the upper housing 51 and the lower housing 52 are connected to the ground potential, only the upper housing 51 can be electrically connected to the substrate 7 via the screw 8; or the upper housing 51 and the lower housing 52 can be electrically connected to the substrate 7.

As explained above, the yarn monitoring device 10 includes the yarn monitoring unit 6 having the upper yarn path guide 65 and the lower yarn path guide 66. The upper charge eliminating member 65a included in the upper yarn path guide 65 is arranged near traveling space R1, and eliminates the electric charge generated by the traveling of the yarn Y. The lower charge eliminating member 66a included in the lower yarn path guide 66 is arranged near traveling space R2, and eliminates the electric charge generated by the traveling of the yarn Y. The upper charge eliminating member 65a is electrically connected to the upper housing 51. The lower charge eliminating member 66a is electrically connected to the lower housing 52.

Consequently, the static electricity generated in the yarn Y, the upper yarn path guide 65, and the lower yarn path guide 66 is guided to the upper housing 51 and the lower housing 52 via the upper charge eliminating member 65a or the lower charge eliminating member 66a. Accordingly, the static electricity can be eliminated from the yarn monitoring device 10. As a result, the effect of the static electricity in the yarn monitoring device 10 can be suppressed. Moreover, because the electrical resistivity of the upper charge eliminating member 65a and the lower charge eliminating member 66a is higher than or equal to the electrical resistivity of the upper housing 51 and the lower housing 52, the static electricity is more effectively guided from the upper charge eliminating member 65a and the lower charge eliminating member 66a to the upper housing 51 and the lower housing 52. As a result, the effects of static electricity can be suppressed more effectively in the yarn monitoring device 10.

Furthermore, probability of occurrence of malfunction and breakage is reduced in the yarn monitoring device 10. Accordingly, a decline in a yarn production volume due to an increase in a yarn production downtime on the spinning machine 1 can be prevented. Moreover, because erroneous detection in the first monitoring section 63 and the second monitoring section 64, which are quality sensors, is reduced in the yarn monitoring device 10, the decline in the yarn production volume and a yarn quality in the spinning machine 1 can be suppressed.

In the yarn monitoring device 10, the upper charge eliminating member 65a is provided in the upper yarn path guide 65 on the upstream side in the traveling direction of the yarn Y with respect to the first holder 61 and the second holder 62. Accordingly, the static electricity from the yarn Y can be eliminated before the yarn Y travels through the traveling space R1. As a result, the effect of the static electricity in the yarn monitoring device 10 can be suppressed.

In the yarn monitoring device 10, the lower charge eliminating member 66a is provided in the lower yarn path guide 66 on the downstream side in the traveling direction of the yarn Y with respect to the first holder 61 and the second holder 62. Accordingly, the static electricity can be eliminated from the yarn Y that is traveling from the traveling space R2 to the outside of the yarn monitoring device 10. As a result, the effect of the static electricity on the spinning machine 1 in which the yarn monitoring device 10 is installed can be suppressed. Moreover, the lower housing 52 can be prevented from becoming electrically charged. Accordingly, accumulation of fly waste can be suppressed, and the decline in measurement accuracy of an amount of light received by the light receiving element in the first monitoring section 63 and the second monitoring section 64 can be suppressed. As a result, because erroneous detection in the first monitoring section 63 and the second monitoring section 64 is reduced, the decline in the yarn production volume and the yarn quality in the spinning machine 1 can be suppressed.

In the yarn monitoring device 10, the upper housing 51 and the lower housing 52 are electrically connected to the ground potential. The upper housing 51 and the lower housing 52 are electrically connected to the ground potential via, for example, the substrate 7 and the screw 8. Accordingly, because the static electricity from the upper housing 51 that is electrically connected to the upper charge eliminating member 65a and the lower housing 52 that is electrically connected to the lower charge eliminating member 66a can be eliminated, the effect of the static electricity in the yarn monitoring device 10 can be suppressed.

In the yarn monitoring device 10, the upper housing 51 contacts the upper charge eliminating member 65a, and the lower housing 52 contacts the lower charge eliminating member 66a. The upper housing 51 contacts the lower housing 52. The lower housing 52 is electrically connected to the substrate 7 via the screw 8. Accordingly, because the static electricity can be eliminated from the upper charge eliminating member 65a and the lower charge eliminating member 66a, the effect of the static electricity in the yarn monitoring device 10 can be suppressed.

In the yarn monitoring device 10, the electrical resistivity of the upper housing 51 and the lower housing 52 is higher than or equal to the electrical resistivity of the screw 8. Accordingly, because the electric charge within the upper housing 51 and the lower housing 52 is more likely to be diffused, the static electricity can be guided more effectively from the upper housing 51 and the lower housing 52 to the substrate 7 via the screw 8. As a result, the effect of the static electricity can be suppressed more effectively in the yarn monitoring device 10.

In the yarn monitoring device 10, both the upper charge eliminating member 65a and the lower charge eliminating member 66a are made of an electrically conductive material. The upper charge eliminating member 65a constitutes the guide surface 65b that guides the yarn Y to the traveling space R1. The lower charge eliminating member 66a constitutes the guide surface 66b that guides the yarn Y from the traveling space R2 to the outside of the yarn monitoring device 10. Accordingly, because the upper charge eliminating member 65a and the lower charge eliminating member 66a that are made of an electrically conductive material directly contact the yarn Y, the static electricity is guided more effectively to the upper housing 51 and the lower housing 52 via the upper charge eliminating member 65a and the lower charge eliminating member 66a. As a result, the effect of the static electricity can be suppressed more effectively in the yarn monitoring device 10. The static electricity is, for example, frictional charging, peeling charge, and the like generated between the upper charge eliminating member 65a, the lower charge eliminating member 66a, and the yarn Y.

The electrical resistivity of the upper charge eliminating member 65a and the lower charge eliminating member 66a is 1.0 × 10⁶ Ω·m or less. Accordingly, the static electricity generated in yarn Y can be eliminated more effectively by the upper charge eliminating member 65a and the lower charge eliminating member 66a.

In the yarn monitoring device 10, the upper charge eliminating member 65a and the lower charge eliminating member 66a can be formed by an electrically conductive resin or an electrically conductive ceramic. Accordingly, the static electricity is guided more effectively to the upper housing 51 and the lower housing 52 via the upper charge eliminating member 65a and the lower charge eliminating member 66a. As a result, the effect of the static electricity can be suppressed more effectively in the yarn monitoring device 10.

In the yarn monitoring device 10, the upper yarn path guide 65 and the lower yarn path guide 66 form an exterior shape of the yarn monitoring device 10. Because of this configuration, a distance between the upper yarn path guide 65 and the lower yarn path guide 66 can be increased. As a result, a maximum amount of time can be secured between a time point at which the yarn Y and the upper yarn path guide 65 contact and a time point at which the yarn Y and the lower yarn path guide 66 contact. Because the yarn Y can be prevented from contacting the upper yarn path guide 65 and the lower yarn path guide 66 in a short time, the decline in the yarn quality can be suppressed.

### Second Embodiment

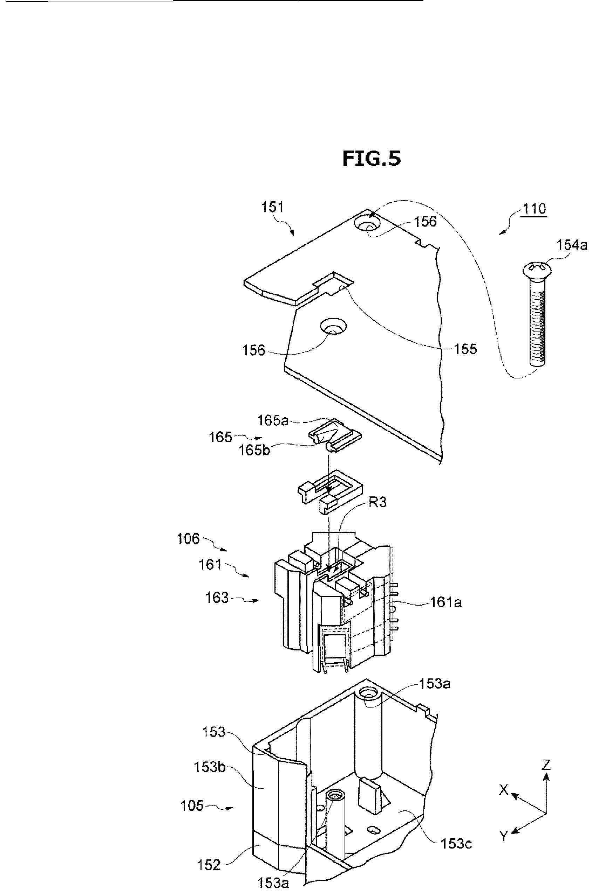

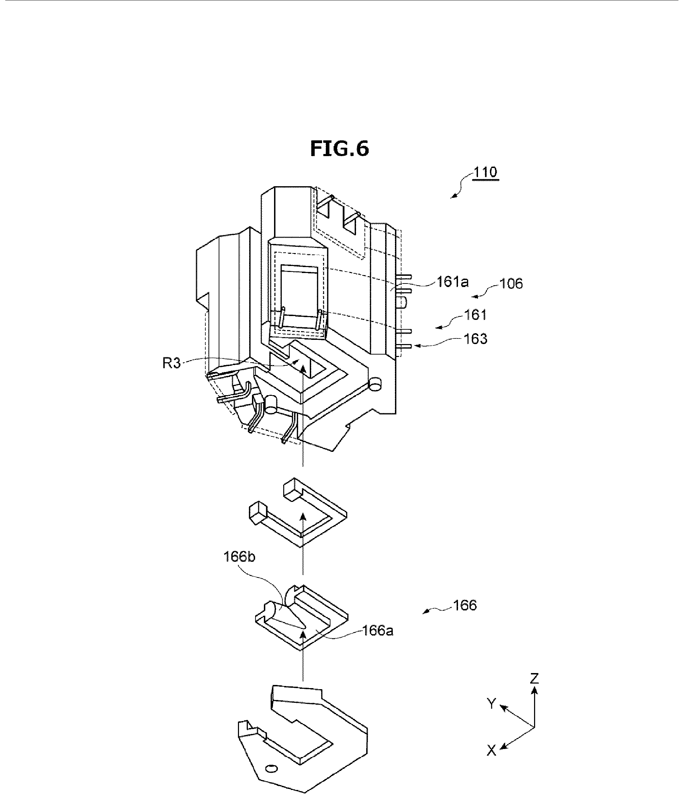

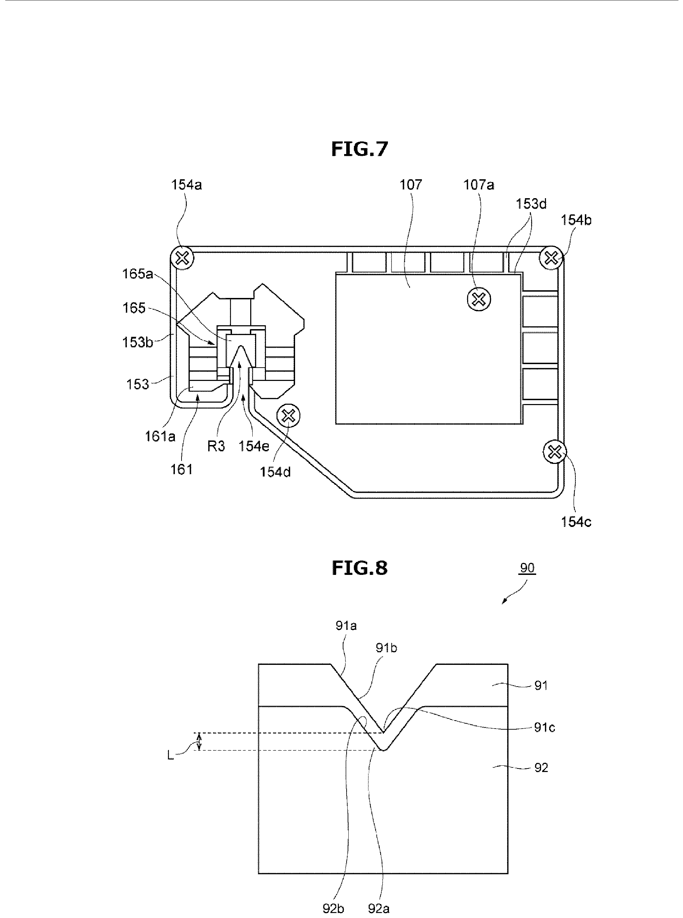

A yarn monitoring device 110 according to a second embodiment is explained below in detail. The yarn monitoring device 110 can be used in the spinning machine 1 of the first embodiment, instead of the yarn monitoring device 10. FIG. 5 is a disassembled perspective view of the yarn monitoring device 110. FIG. 6 is a disassembled perspective view of the yarn monitoring device 110 from a different angle. FIG. 7 is a plan view of the yarn monitoring device 110. In FIG. 7, a later-explained top plate 151 is omitted. As shown in FIGS. 5, 6, and 7, the yarn monitoring device 110 includes a housing 105, a yarn monitoring unit 106, and a substrate 107. The yarn monitoring unit 106 and the substrate 107 are arranged inside the housing 105.

The housing 105 includes a main body section 153, the top plate (connecting member, upstream connecting member) 151, and a bottom plate (connecting member, downstream connecting member) 152. The housing 105 further includes a screw 154a (second electrically conductive member) and three other screws 154b, 154c, and 154d (see FIG. 7).

As shown in FIG. 5, the main body section 153 is a cylindrical member having a bottom but the top open. A cylindrical part 153b (first electrically conductive member) of the main body section 153 extends along the traveling direction of yarn Y, and surrounds the yarn monitoring unit 106. A notch 153e corresponding to a later-explained traveling space R3 is formed on the main body section 153. The main body section 153 includes a bottom part 153c that is provided so as to block an opening on the downstream side of the traveling direction of the yarn Y in the cylindrical part 153b. The main body section 153 is formed, for example, by an electrically conductive resin. The main body section 153 includes a plurality of hole portions 153a. The main body section 153 includes a supporting part 153d (first electrically conductive member) that contacts the cylindrical part 153b. The supporting part 153d supports the substrate 107.

The top plate 151 is provided on the main body section 153 so as to close an opening of the main body section 153. The top plate 151 is, for example, a plate member that extends orthogonal to the traveling direction of the yarn Y. The top plate 151 fixes an upper yarn path guide 165 to a holder 161. The top plate 151 is arranged facing the bottom part 153c of the main body section 153 in the traveling direction of the yarn Y. The top plate 151 is made of an electrically conductive material, such as metal. The electrical resistivity of the top plate 151 is, for example, higher than or equal to the electrical resistivity of a later-explained screw 107a. An opening part 155 and a plurality of round holes 156 are provided on the top plate 151. A portion of the upper yarn path guide 165 (an upper part of a later-explained upper charge eliminating member 165a) is engaged into the opening part 155. Each of the round holes 156 is provided at a position that corresponds to each of the hole portions 153a on the main body section 153. The top plate 151 is fixed to the main body section 153 by screwing in four screws 154a to 154d into each of the round holes 156 and each hole portion 153a on the main body section 153. In other words, the top plate 151 contacts the screws 154a to 154d.

The bottom plate 152 is provided at the bottom part 153c on the downstream side of the traveling direction of the yarn Y in the main body section 153. The bottom part 153c is interposed between the bottom plate 152 and the yarn monitoring unit 106. The bottom plate 152 is arranged facing the top plate 151 in the traveling direction of the yarn Y. The bottom plate 152 is, for example, a plate member that extends orthogonal to the traveling direction of the yarn Y. The bottom plate 152 is made of an electrically conductive material, such as metal. The electrical resistivity of the bottom plate 152 is higher than or equal to the electrical resistivity of the later-explained screw 107a. The bottom plate 152 is electrically connected to the screw 154a, and, for example, contacts the screw 154a.

As shown in FIGS. 5 and 6, the yarn monitoring unit 106 includes the holder 161, a monitoring section 163, an upper yarn path guide (yarn path guide, upstream yarn path guide) 165, and a lower yarn path guide (yarn path guide, downstream yarn path guide) 166. The yarn monitoring unit 106 is arranged between the top plate 151 and the bottom plate 152.

A main body 161a of the holder 161 is provided with a traveling space R3 through which the yarn Y travels. The traveling space R3 is a space that extends along the traveling space of the yarn Y. The traveling space R3 is open on one side (front side) in a longitudinal direction. In other words, the traveling space R3 is a space through which the traveling yarn Y passes, and it is formed such that it extends from upstream to downstream and has a groove shape that is open on the front side. When seen from the traveling direction of the yarn Y, the traveling space R3 is formed in a U-shape. Alternatively, the traveling space R3 can have a shape of English letter V when seen from the traveling direction of the yarn Y.

The monitoring section 163 is attached to the main body 161a of the holder 161. The monitoring section 163 monitors the state of the yarn Y that is traveling through the traveling space R3. The monitoring section 163 includes, for example, a not-shown light emitting element and a not-shown light receiving element. The light emitting element and the light receiving element are mounted inside the holder 161, and are supported by the main body 161a. The monitoring section 163 monitors the state of the yarn Y by projecting light from the light emitting element onto the yarn Y that is traveling through the traveling space R3, and receiving the light reflected and / or transmitted from the yarn Y by using the light receiving element. An LED and the like, for example, can be used as the light emitting element. A photodiode and the like, for example, can be used as the light receiving element.

The upper yarn path guide 165 is provided in the holder 161 at the upstream side of the yarn in the traveling direction with respect to the holder 161. In the present embodiment, the upper yarn path guide 165 is arranged between the holder 161 and the top plate 151. The upper yarn path guide 165 is, for example, a rectangular plate member in which a grooved part (a groove having shape of English letter V) that extends in a thickness direction (traveling direction of the yarn Y) and opens at the front side is formed. The upper yarn path guide 165 includes an upper charge eliminating member (charge eliminating member, upstream charge eliminating member) 165a that includes a grooved part. A side surface (equivalent to plate thickness) of the grooved part in the upper charge eliminating member 165a is a guide surface 165b that guides the yarn Y. In other words, the upper charge eliminating member 165a forms the guide surface 165b that guides the yarn Y in the traveling space R3. In an example shown in FIG. 4, similar to the guide surface 65b, the guide surface 165b always contacts the yarn Y, and regulates the traveling path of the yarn Y. Accordingly, the guide surface 165b guides the traveling yarn Y into the traveling space R3. The traveling yarn Y is pulled backward and pressed against the guide surface 165b that is a bottom of the grooved part. In this manner, the position of the yarn y is fixed. Furthermore, the grooved part formed on the upper yarn path guide 165 can have a shape of English letter U when seen from the traveling direction of the yarn Y.

The upper charge eliminating member 165a is, for example, made of an electrically conductive material. The entire upper yarn path guide 165 can be the upper charge eliminating member 165a, or only a portion of the upper yarn path guide 165 (the portion forming the guide surface 165b) can be the upper charge eliminating member 165a. The upper charge eliminating member 165a is positioned near an upper end (entrance) of the traveling space R3, and is arranged adjacent to the traveling space R3. In the present embodiment, the upper charge eliminating member 165a is a part of the upper yarn path guide 165, and is arranged at a position where it contacts the traveling yarn Y. The material from which the upper charge eliminating member 165a is made is determined according to the compatibility of that material with the material of the yarn Y and a traveling speed of the yarn Y. The upper charge eliminating member 165a, for example, can be formed by a metal, such as stainless steel or copper, or can be formed by an electrically conductive resin or an electrically conductive ceramic. The electrical resistivity of the upper charge eliminating member 165a is, for example, higher than or equal to the electrical resistivity of the top plate 151. The electrical resistivity of the upper charge eliminating member 165a is, for example, 1.0 × 10⁶ Ω·m or less. Furthermore, the upper charge eliminating member 165a can be arranged at a position where it does not contact the traveling yarn Y. In other words, the upper charge eliminating member 165a can be provided in a portion other than the guide surface 165b. In such a configuration, it is sufficient that the upper charge eliminating member 165a is arranged near the yarn Y such that the static electricity can be eliminated from the yarn Y, for example, with a small gap between itself and the yarn Y.

The lower yarn path guide 166 is provided in the holder 161 at the downstream side of the yarn Y in the traveling direction with respect to the holder 161. In the present embodiment, the lower yarn path guide 166 is arranged between the holder 161 and the bottom plate 152. The lower yarn path guide 166 is, for example, a rectangular plate member in which a grooved part (a groove having shape of English letter V) that extends in a thickness direction (traveling direction of the yarn Y) and opens at the front side is formed. The configuration of the lower yarn path guide 166 is the same as that of the upper yarn path guide 165, and includes a lower charge eliminating member (charge eliminating member, downstream charge eliminating member) 166a that includes a grooved part. A side surface (equivalent to plate thickness) of the grooved part in the lower charge eliminating member 166a is a guide surface 166b that guides the yarn Y. In other words, the lower charge eliminating member 166a forms the guide surface 166b that guides the yarn Y from the traveling space R3 to the outside of the yarn monitoring device 110. In an example shown in FIG. 4, similar to the guide surface 66b, the guide surface 166b always contacts the yarn Y, and regulates the traveling path of the yarn Y. Accordingly, the guide surface 166b guides the traveling yarn Y from the traveling space R3 to the outside of the yarn monitoring device 110. The traveling yarn Y is pulled backward and pressed against the guide surface 166b that is a bottom of the grooved part. In this manner, the position of the yarn y is fixed. Furthermore, the grooved part formed on the lower yarn path guide 166 can have a shape of English letter U when seen from the traveling direction of the yarn Y. The lower yarn path guide 166 is arranged such that the upstream side and downstream side of the lower yarn path guide 166 are inverted vertically with respect to the upper yarn path guide 165. In other words, the upper yarn path guide 165 and the lower yarn path guide 166 are arranged facing each other in a reversed manner in the up-down direction.

The lower charge eliminating member 166a is, for example, made of an electrically conductive material. The entire lower yarn path guide 166 can be the lower charge eliminating member 166a, or only a portion of the lower yarn path guide 166 (the portion forming the guide surface 166b) can be the lower charge eliminating member 166a. The lower charge eliminating member 166a is positioned near a lower end (exit) of the traveling space R3, and is arranged adjacent to the traveling space R3. In the present embodiment, the lower charge eliminating member 166a is a part of the lower yarn path guide 166, and is arranged at a position so as to come in contact with the traveling yarn Y. The material from which the lower charge eliminating member 166a is made is determined according to the compatibility of that material with the material of the yarn Y and a traveling speed of the yarn Y. The lower charge eliminating member 166a, for example, can be formed by a metal, such as stainless steel or copper, or can be formed by an electrically conductive resin or an electrically conductive ceramic. The electrical resistivity of the lower charge eliminating member 166a is higher than or equal to the electrical resistivity of the bottom plate 152. The electrical resistivity of the lower charge eliminating member 166a is, for example, 1.0 × 10⁶ Ω·m or less. Furthermore, the lower charge eliminating member 166a can be arranged at a position so as not to come in contact with the traveling yarn Y. In other words, the lower charge eliminating member 166a can be provided in a portion other than the guide surface 166b. In such a configuration, it is sufficient that the lower charge eliminating member 166a is arranged near the yarn Y such that the static electricity can be eliminated from the yarn Y, for example, with a small gap between itself and the yarn Y.

The substrate 107 transmits information acquired by the monitoring section 163 to the outside of the yarn monitoring device 110. The substrate 107 is arranged inside the housing 105. The substrate 107 extends on a plane that is orthogonal to the traveling direction of the yarn Y inside the housing 105. A hole portion is formed on the substrate 107. The substrate 107 is fixed to the supporting part 153d by screwing in the screw 107a (first electrically conductive member) into the hole portion.

The upper charge eliminating member 165a is, for example, electrically connected to the ground potential. In the present embodiment, the upper charge eliminating member 165a contacts the top plate 151, and is electrically connected to the top plate 151. The top plate 151 is electrically connected to the substrate 107 via the screw 107a. The substrate 107 is electrically connected to the ground potential. The upper charge eliminating member 165a is electrically connected to the outside of the yarn monitoring device 110. Accordingly, the electric charge removed from the yarn Y by the upper charge eliminating member 165a is guided to the outside of the yarn monitoring device 110. However, because the upper charge eliminating member 165a should preferably be electrically connected to the ground potential, the upper charge eliminating member 165a can be connected to the ground potential (earth) by using a path other than that explained above.

The lower charge eliminating member 166a is, for example, electrically connected to the ground potential. In the present embodiment, the lower charge eliminating member 166a contacts the bottom plate 152, and is electrically connected to the bottom plate 152. The bottom plate 152 is electrically connected to the top plate 151 via the screw 154a. The top plate 151 is electrically connected to the ground potential as explained above. In other words, the lower charge eliminating member 166a is electrically connected to the outside of the yarn monitoring device 110. Accordingly, the electric charge eliminated from the yarn Y by the lower charge eliminating member 166a is then guided to the outside of the yarn monitoring device 110. However, because the lower charge eliminating member 166a should preferably be electrically connected to the ground potential, the lower charge eliminating member 166a can be connected to the ground potential (earth) by using a path other than that explained above. Moreover, because the top plate 151 and the bottom plate 152 should preferably be electrically connect to the ground potential, the bottom plate 152 can be electrically connected to the substrate 107 via the main body section 153 and the screw 154a.

In the yarn monitoring device 110 explained above, the upper charge eliminating member 165a is electrically connected to the top plate 151. The lower charge eliminating member 166a contacts the bottom plate 152, and is electrically connected to the bottom plate 152. The bottom plate 152 is electrically connected to the top plate 151 via the screw 154a. The top plate 151 is electrically connected to the substrate 107 via the supporting part 153d and the screw 107a. The substrate 107 is electrically conductive to the outside of the yarn monitoring device 110, and is electrically connected to the ground potential. Accordingly, because the static electricity is eliminated from the yarn monitoring device 110, the effect of the static electricity in the yarn monitoring device 110 can be suppressed.

Similar to the yarn monitoring device 10 according to the first embodiment, the yarn monitoring device 110 according to the second embodiment includes the yarn monitoring unit 106 that includes the upper yarn path guide 165 and the lower yarn path guide 166. The upper charge eliminating member 165a in the upper yarn path guide 165 and the lower charge eliminating member 66a in the lower yarn path guide 166 are arranged adjacent to the traveling space R3. The upper charge eliminating member 165a is electrically connected to the top plate 151. The lower charge eliminating member 166a is electrically connected to the bottom plate 152.

Therefore, the static electricity generated in the yarn Y, the upper yarn path guide 165, and the lower yarn path guide 166 is guided to the top plate 151 and the bottom plate 152 via the upper charge eliminating member 165a or the lower charge eliminating member 166a. Accordingly, the static electricity can be eliminated from the yarn monitoring device 110. As a result, the effect of the static electricity in the yarn monitoring device 110 can be suppressed. Moreover, because the electrical resistivity of the upper charge eliminating member 165a and the lower charge eliminating member 166a is higher than or equal to the electrical resistivity of the top plate 151 and the bottom plate 152, the static electricity is guided more effectively from the upper charge eliminating member 165a and the lower charge eliminating member 166a to the top plate 151 and the bottom plate 152. As a result, the effect of the static electricity can be suppressed more effectively in the yarn monitoring device 110. Moreover, malfunction and breakage incidences are reduced in the yarn monitoring device 110. Accordingly, an amount of time during which the yarn traveling is stopped in a device in which the yarn monitoring device 110 is mounted can be suppressed. Moreover, erroneous detection by the monitoring section 163, which is the yarn quality sensor, in the yarn monitoring device 110 can be suppressed. Furthermore, the yarn monitoring device 110 according to the second embodiment demonstrates other similar effects and advantages as that of the yarn monitoring device 10 according to the first embodiment.

### Modifications

Embodiments of the present invention are explained above; however, the present invention is not limited to the embodiments explained above. Various modifications of the embodiments of the present invention are acceptable as long as the corresponding modification does not deviate from the scope of the claims.

In the above embodiments, the upper charge eliminating members 65a and 165a, and the lower charge eliminating members 66a and 166a, which are the charge eliminating members, are a part of the upper yarn path guides 65 and 165, and the lower yarn path guides 66 and 166, respectively, and such charge eliminating members are arranged so as to contact the traveling yarn Y. However, in each yarn path guide, it is sufficient that the charge eliminating member is arranged near the yarn Y such that the static electricity can be eliminated from the yarn Y.

FIG. 8 is a diagram showing a yarn path guide 90 according to a modification. In the above embodiments, instead of the upper yarn path guides 65 and 165, and the lower yarn path guides 66 and 166, the yarn path guide 90 can be applied in the yarn monitoring devices 10 and 110. In the present modification, the yarn path guide 90 includes a guide main body 91 and a charge eliminating body 92 that is attached to the guide main body 91.

The guide main body 91 includes a guide surface 91a that guides the yarn Y. In the guide main body 91, for example, a grooved part 91b having shape of English letter V is formed on a rectangular plate member. The grooved part 91b is demarcated by the guide surface 91a. The guide main body 91 is made from, for example, ceramic. The charge eliminating body 92 is, for example, made of an electrically conductive material. The material from which the charge eliminating body 92 is made is determined according to the compatibility of that material with the material of the yarn Y and a traveling speed of the yarn Y. The charge eliminating body 92, for example, can be formed by a metal, such as stainless steel or copper, or can be formed by an electrically conductive resin or an electrically conductive ceramic. The charge eliminating body 92 includes a charge eliminating member 92a that is arranged along the grooved part 91b of the guide main body 91. The charge eliminating member 92a is arranged on the guide main body 91 adjacent to the guide surface 91a. Specifically, the charge eliminating member 92a is arranged adjacent to a bottom part 91c of the grooved part 91b. In the example shown in FIG. 8, the grooved part 92b having shape of English letter V that is formed by the charge eliminating member 92a is provided on the charge eliminating body 92. In the charge eliminating member 92a of the charge eliminating body 92, the grooved part 92b is arranged on the guide main body 91 so as to position along the grooved part 91b.

Moreover, even when the guide surface 91a on which the static electricity is generated is separated from the charge eliminating member 92a, the static electricity is flown from the guide surface 91a to the charge eliminating member 92a. Alternatively, the charge eliminating member 92a can be formed by performing plating on the guide main body 91. Moreover, in FIG. 8, a distance L between the charge eliminating member 92a and the bottom part 91c is n millimeters (mm). For example, the distance L can be 0.7 mm, or can be 0.4 mm. Furthermore, the distance L can be 0 mm. In other words, when the yarn path guide 90 is seen from the traveling direction of the yarn Y, the bottom part 91c and the charge eliminating member 92a can be overlapping.

According to the above configuration, because the static electricity is guided from the guide surface 91a to the charge eliminating body 92 via the charge eliminating member 92a, the static electricity can be eliminated from the guide surface 91a. As a result, the effect of the static electricity in the yarn monitoring devices 10 and 110 can be suppressed. Moreover, by attaching the charge eliminating body 92 on the guide main body 91, the static electricity can be eliminated from the guide surface 91a without modifying the configuration of the guide main body 91. For example, a static electricity eliminating function can be imparted to the yarn monitoring devices 10 and 110 simply by attaching (adding) the charge eliminating body 92 while using the existing guide main body 91 that is made of an insulating material that does not have the static electricity eliminating function. Moreover, for example, the static electricity can be eliminated while using the guide main body 91 that is made from the existing wear-resistant material. Moreover, even when the yarn has moved to front, back, left, or right side (above, below, left, or right in FIG. 8) of the grooved part 91b while the yarn Y is traveling, the static electricity can be flown to the charge eliminating member 92a even when the static electricity is generated anywhere in the grooved part 91b by providing the charge eliminating member 92a along the grooved part 91b.

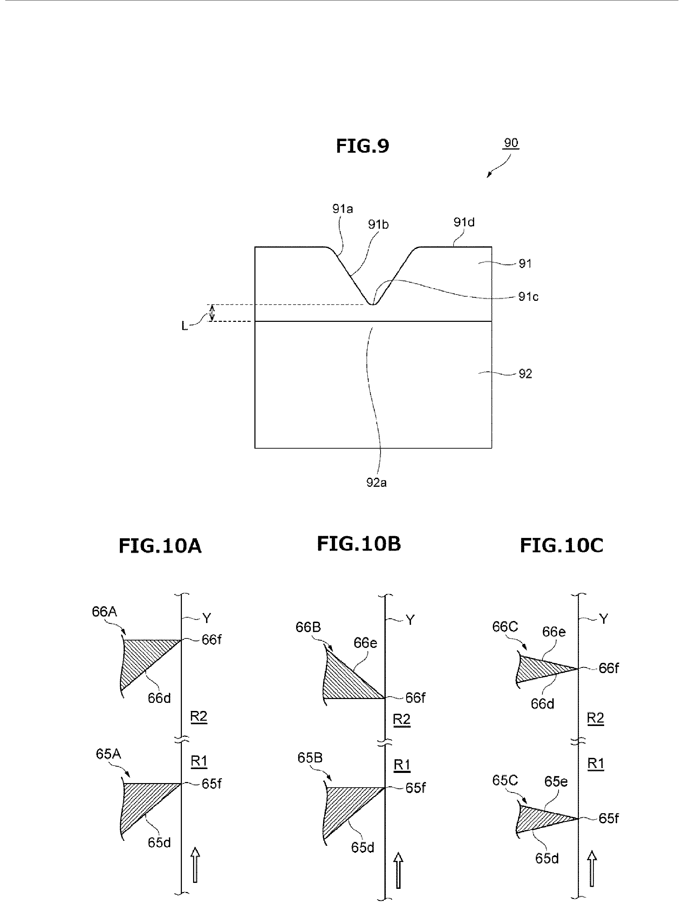

In the configuration explained above, it is acceptable if the charge eliminating body 92 does not include the grooved part 92b. FIG. 9 is a diagram showing the yarn path guide 90 according to another modification. In an example shown in FIG. 9, the charge eliminating member 92a of the charge eliminating body 92 extends linearly along an edge 91d on which an opening of the grooved part 91b having shape of English letter V is formed. The charge eliminating member 92a is arranged on the guide main body 91 along a direction in which the grooved part 91b extends. Moreover, in FIG. 9, a distance L between the charge eliminating member 92a and the bottom part 91c is n millimeters (mm). For example, the distance L can be 0.7 mm, or can be 0.4 mm. Furthermore, the distance L can be 0 mm. In other words, when the yarn path guide 90 is seen from the traveling direction of the yarn Y, the bottom part 91c and the charge eliminating member 92a can be overlapping.

In the upper yarn path guides 65 and 165, and the lower yarn path guides 66 and 166 according to the first embodiment, the second embodiment, and the modifications explained above, the guide surfaces 65b, 66b, 165b, and 166b always contact the yarn Y. A combination of an upper yarn path guide and a lower yarn path guide according to the modification is explained below with reference to FIGS. 10A, 10B, and 10C.

FIG. 10A is a cross-sectional view of an upper yarn path guide 65A and a lower yarn path guide 66A according to a first modification. As shown in FIG. 10A, the upper yarn path guide 65A and the lower yarn path guide 66A are of the same shape, and are provided by arranging in the same direction with respect to the yarn Y to be guided. The upper yarn path guide 65A includes a tip end edge 65f that guides the traveling yarn Y, and an upstream side inclined surface 65d (inclined surface) that extends towards an upstream side in the traveling direction of the tip end edge 65f. The upstream side inclined surface 65d is formed by an R surface. The upstream side inclined surface 65d is connected to the tip end edge 65f, and inclines away from the traveling space R1. However, the upstream side inclined surface 65d may be other than a linear-shaped surface. In the upper yarn path guide 65A, the tip end edge 65f that protrudes toward the traveling space R1 contacts the yarn Y; however, the upstream side inclined surface 65d is recessed from the tip end edge 65f with respect to the traveling space R1 and does not contact the yarn Y. The lower yarn path guide 66A includes a tip end edge 66f that guides the traveling yarn Y, and an upstream side inclined surface 66d (inclined surface) that extends towards an upstream side in the traveling direction of the tip end edge 66f. The upstream side inclined surface 66d is connected to the tip end edge 66f, and inclines away from the traveling space R2. In the lower yarn path guide 66A, the tip end edge 66f that protrudes toward the traveling space R2 contacts the yarn Y, and the upstream side inclined surface 66d is recessed from the tip end edge 66f with respect to the traveling space R2 and does not contact the yarn Y. The tip end edge 65f of the upper yarn path guide 65A and the tip end edge 66f of the lower yarn path guide 66A are arranged in a straight line (yarn path) along the traveling direction of the yarn Y. When passing through the upper yarn path guide 65A and the lower yarn path guide 66A, the yarn Y contacts the upper yarn path guide 65A and the lower yarn path guide 66A only at a downstream end portion in a thickness direction of the respective yarn path guide (the tip end edge 65f and the tip end edge 66f).

FIG. 10B is a cross-sectional view of an upper yarn path guide 65B and a lower yarn path guide 66B according to a second modification. As shown in FIG. 10B, the upper yarn path guide 65B and the lower yarn path guide 66B are of the same shape, and are provided by arranging in an opposite direction of the traveling direction of the yarn Y, with respect to the yarn Y to be guided. Because a configuration of the upper yarn path guide 65B is the same as that of the upper yarn path guide 65A, explanation thereof is omitted. The lower yarn path guide 66B includes the tip end edge 66f that guides the traveling yarn Y, and a downstream side inclined surface 66e (inclined surface) that extends towards a downstream side in the traveling direction of the tip end edge 66f. The downstream side inclined surface 66e is connected to the tip end edge 66f, and inclines away from the traveling space R2. In the lower yarn path guide 66B, the tip end edge 66f that protrudes toward the traveling space R2 contacts the yarn Y; however, the downstream side inclined surface 66e is recessed from the tip end edge 66f with respect to the traveling space R2 and does not contact the yarn Y. The tip end edge 65f of the upper yarn path guide 65B and the tip end edge 66f of the lower yarn path guide 66B are arranged in a straight line (yarn path) along the traveling direction of the yarn Y. When passing through the upper yarn path guide 65B, the yarn Y comes in contact only with the upper yarn path guide 65B only at a downstream end portion (the tip end edge 65f) in a thickness direction of the upper yarn path guide 65B, and contacts the lower yarn path guide 66B only at the upstream end portion (the tip end edge 66f) of the thickness direction of the lower yarn path guide 66B.

FIG. 10C is a cross-sectional view of an upper yarn path guide 65C and a lower yarn path guide 66C according to a third modification. As shown in FIG. 10A, the upper yarn path guide 65A and the lower yarn path guide 66C are of the same shape, and are provided by arranging in the same direction with respect to the yarn Y to be guided. The upper yarn path guide 65C includes the tip end edge 65f that guides the traveling yarn Y, and the upstream side inclined surface 65d and the downstream side inclined surface 65e that extend toward the upstream side and the downstream side in the traveling direction of the tip end edge 65f, respectively. An inclination angle of the upstream side inclined surface 65d and the downstream side inclined surface 65e (inclined surface) can be the same as that of the upstream side inclined surface 65d and the downstream side inclined surface 65e according to the first modification and the second modification explained above, or can be different from that of the upstream side inclined surface 65d and the downstream side inclined surface 65e according to the first modification and the second modification explained above. In an example shown in FIG. 10C, the upstream side inclined surface 65d and the downstream side inclined surface 65e incline at a larger angle with respect to the traveling direction of the yarn Y than that of the upstream side inclined surface 65d and the downstream side inclined surface 65e according to the first modification and the second modification. In the upper yarn path guide 65C, the tip end edge 65f that protrudes toward the traveling space R1 contacts the yarn Y; however, the upstream side inclined surface 65d and the downstream side inclined surface 65e are recessed from the tip end edge 65f with respect to the traveling space R1 and does not contact the yarn Y. Similarly, the lower yarn path guide 66C includes the tip end edge 66f that guides the traveling yarn Y, and the upstream side inclined surface 66d and the downstream side inclined surface 66e that extend toward the upstream side and the downstream side in the traveling direction of the tip end edge 66f, respectively. The inclination angle of the upstream side inclined surface 66d and the downstream side inclined surface 66e can be said to be the same as that of the upstream side inclined surface 65d and the downstream side inclined surface 65e of the upper yarn path guide 65C. Even in the lower yarn path guide 66C, the tip end edge 66f that protrudes toward the traveling space R2 contact the yarn Y; however, the upstream side inclined surface 66d and the downstream side inclined surface 66e are recessed from the tip end edge 66f with respect to the traveling space R2 and does not contact the yarn Y. The tip end edge 65f of the upper yarn path guide 65C and the tip end edge 66f of the lower yarn path guide 66C are arranged in a straight line (yarn path) along the traveling direction of the yarn Y. When passing through the upper yarn path guide 65C and the lower yarn path guide 66C, the yarn Y comes in contact only with the upper yarn path guide 65C and the lower yarn path guide 66C only at a middle part in a thickness direction of the respective yarn path guide (the tip end edge 65f and the tip end edge 66f).

In each configuration explained above, the upper yarn path guides 65A, 65B, and 65C, and the lower yarn path guides 66A, 66B, and 66C guide the yarn Y at two points of the tip end edge 65f and the tip end edge 66f, respectively. For example, because a contact area between the yarn Y, the upper yarn path guide 65, and the lower yarn path guide 66 is reduced compared to a configuration in which the yarn Y is guided by the guide surfaces 65b, 165b, 66b, and 166b, generation of the static electricity can be suppressed in the yarn Y, the upper yarn path guides 65A, 65B, 65C, and the lower yarn path guides 66A, 66B, 66C.

In the first embodiment, the second embodiment, and the modifications, a constituent requirement or a feature explained as "higher than or equal to" with respect to a magnitude correlation of the electrical resistivity of various charge eliminating members, various connecting members, and various electrically conductive members can be replaced with a constituent requirement or a feature of "higher than". The electrical resistivity of at least one of the charge eliminating members can be higher than the electrical resistivity of at least one of the electrically connected connecting members to be compared. The electrical resistivity of at least one of the connecting members can be higher than the electrical resistivity of at least one of the electrically connected electrically conductive members to be compared. Because there is a difference in the electrical resistivity, ease of a diffusion of the electric charge explained above is enhanced, and as a result, an effect of eliminating the static electricity from the yarn is also enhanced.

In the second embodiment explained above, the top plate 151 is a member that is positioned at the utmost upstream in the traveling direction of the yarn Y, and the bottom plate 152 is a member that is positioned at the utmost downstream in the traveling direction of the yarn Y; however, the configuration is not limited thereto. For example, the top plate 151 can be arranged at a downstream of the upper yarn path guide 165 in the traveling direction of the yarn Y. Moreover, the bottom plate 152 can be arranged at an upstream of the lower yarn path guide 166 in the traveling direction of the yarn Y.

In the second embodiment explained above, the cylindrical part 153b and the supporting part 153d can be electrically conductive members. The electrical resistivity of the top plate 151 can be, for example, higher than or equal to the resistivity of the cylindrical part 153b and the supporting part 153d. The electrical resistivity of the bottom plate 152 can be, for example, higher than or equal to the electrical resistivity of the cylindrical part 153b and the supporting part 153d. In such a configuration, the top plate 151 is electrically connected to the substrate 107 via the cylindrical part 153b, the supporting part 153d, and the screw 107a. In such a configuration, the cylindrical part 153b, the supporting part 153d, and the screw 107a are equivalent to the first electrically conductive member.

Moreover, the bottom plate 152 is electrically connected to the substrate 107 via the cylindrical part 153b, the supporting part 153d, and the screw 107a. Because of such electrical connection, the static electricity is less likely to accumulate on the bottom plate 152, and therefore, the effect of the static electricity on the yarn monitoring device 110 can be suppressed. A similar electrical connection can be provided for the top plate 151.

The screw 107a can be an electric insulating member. In this case, the top plate 151 and the bottom plate 152 can be electrically connected via the cylindrical part 153b. In such a configuration, the cylindrical part 153b is equivalent to the second electrically conductive member.

In the first embodiment, the second embodiment, and the modifications, the yarn monitoring devices 10 and 110 can be used in various types of yarn-related devices other than the spinning machine 1.

### Test Example

As a test example, a prototype of a device that has the same configuration as that of the yarn monitoring device 10 according to the embodiments explained above was manufactured, and an effect of a yarn path guide that is made of an electrically conductive material was confirmed. Moreover, as a comparison example, a yarn path guide (that does not include a charge eliminating member) made of a conventionally used ceramic material, and a cotton yarn was used. In a yarn monitoring device of the comparison example, during the monitoring of the yarn made of cotton (cotton Ne40), a detection value within a predetermined light intensity range was detected in a region corresponding to a short foreign substance. On the other hand, in the yarn monitoring device of the comparison example, during the monitoring of the yarn made of wool (wool Nm48), not only the detection value within the predetermined light intensity range but also a detection value of an extremely small light amount (dark) was detected in a region corresponding to a short foreign substance. In the case of wool, the static electricity was generated in the yarn, and it was assumed that such an abnormal value is detected because of the effect of the static electricity. In the yarn monitoring device according to the test example, even during the monitoring of the yarn made of wool (wool Nm48), the detection value within the predetermined light intensity range was detected, but the detection value of the extremely small light amount (dark) was not detected in the region corresponding to a short foreign substance. The static electricity generated in the yarn made of the wool was appropriately eliminated by the charge eliminating member and the connecting member.

According to one aspect of the present invention, a yarn monitoring device that monitors a state of a yarn that travels in a traveling direction through a traveling space includes a housing; and a yarn monitoring unit arranged inside the housing. The yarn monitoring unit includes a monitoring section that monitors a state of the yarn that travels through the traveling space; a yarn path guide that guides the traveling yarn; and a holder that houses the monitoring section and the yarn path guide. The yarn path guide is arranged adjacent to the traveling space, and includes a charge eliminating member that eliminates an electric charge generated by the traveling of the yarn. The housing includes a connecting member that is electrically connected to the charge eliminating member. An electrical resistivity of the charge eliminating member is higher than or equal to an electrical resistivity of the connecting member.

According to the above yarn monitoring device, static electricity generated in the yarn path guide and the yarn is guided to the connecting member via the charge eliminating member. Accordingly, the static electricity can be eliminated from the yarn monitoring device. As a result, an effect of static electricity in the yarn monitoring device can be suppressed. Moreover, the electrical resistivity of the charge eliminating member is higher than or equal to the electrical resistivity of the connecting member. Accordingly, because the electric charge can be easily diffused in the connecting member, the static electricity is guided more effectively from the charge eliminating member to the connecting member. As a result, the effect of the static electricity can be suppressed more effectively in the yarn monitoring device.

The yarn path guide can be an upstream yarn path guide that is provided on an upstream side in the traveling direction of the yarn with respect to the holder, and the charge eliminating member can be an upstream charge eliminating member that is provided in the upstream yarn path guide. According to this configuration, the static electricity can be eliminated from the yarn that is traveling through the traveling space. As a result, the effect of the static electricity can be suppressed in the yarn monitoring device.

The yarn path guide can be a downstream yarn path guide that is provided on a downstream side in the traveling direction of the yarn with respect to the holder, and the charge eliminating member can be a downstream charge eliminating member that is provided in the downstream yarn path guide. With such a configuration, the static electricity can be eliminated from the yarn that is traveling to the outside of the yarn monitoring device. As a result, the effect of the static electricity in an external device in which the yarn monitoring device is installed can be suppressed.

The yarn path guide can include an upstream yarn path guide that is provided on an upstream side in the traveling direction of the yarn with respect to the holder and a downstream yarn path guide that is provided on a downstream side in the traveling direction of the yarn with respect to the holder, and the charge eliminating member can include an upstream charge eliminating member provided in the upstream yarn path guide, and a downstream charge eliminating member provided in the downstream yarn path guide. According to this configuration, the static electricity can be eliminated from the yarn that is traveling through the traveling space. Furthermore, the static electricity can be eliminated from the yarn that is traveling to the outside of the yarn monitoring device. The effect of the static electricity in the external device in which the yarn monitoring device is installed can be suppressed while suppressing the effect of the static electricity in the yarn monitoring device.

The connecting member is electrically connected to a ground potential. With such a configuration, because the static electricity is eliminated from the charge eliminating member via the connecting member that is electrically connected to the ground potential, the effect of the static electricity in the yarn monitoring device can be suppressed.

The above yarn monitoring device includes a first electrically conductive member that is arranged in the housing; and a substrate that sends information acquired by the monitoring section to the outside of the yarn monitoring device, and is electrically connected to a ground potential. The connecting member is electrically connected to the substrate via the first electrically conductive member. With such a configuration, because the static electricity is eliminated from the connecting member via the first electrically conductive member and the substrate, the effect of the static electricity in the yarn monitoring device can be suppressed.

The above yarn monitoring device can include a first electrically conductive member that is arranged in the housing; and a substrate that sends information acquired by the monitoring section to the outside of the yarn monitoring device, and is electrically connected to a ground potential. The yarn path guide can include an upstream yarn path guide that is provided on an upstream side in the traveling direction of the yarn with respect to the holder and a downstream yarn path guide that is provided on a downstream side in the traveling direction of the yarn with respect to the holder. The charge eliminating member can include an upstream charge eliminating member provided in the upstream yarn path guide, and a downstream charge eliminating member provided in the downstream yarn path guide. The connecting member can include an upstream connecting member that is connected to the upstream charge eliminating member, and a downstream connecting member that is connected to the downstream charge eliminating member. The upstream connecting member can contact the downstream connecting member, and can is electrically connected to the downstream connecting member. The upstream connecting member or the downstream connecting member can be electrically connected to the substrate via the first electrically conductive member. With such a configuration, because the static electricity is eliminated from the upstream connecting member and the downstream connecting member via the first electrically conductive member and the substrate, the effect of the static electricity in the yarn monitoring device can be suppressed.

The above yarn monitoring device can include a first electrically conductive member and a second electrically conductive member that are arranged in the housing; and a substrate that sends information acquired by the monitoring section to the outside of the yarn monitoring device, and is electrically connected to a ground potential. The yarn path guide can include an upstream yarn path guide that is provided on an upstream side in the traveling direction of the yarn with respect to the holder and a downstream yarn path guide that is provided on a downstream side in the traveling direction of the yarn with respect to the holder. The charge eliminating member can include an upstream charge eliminating member provided in the upstream yarn path guide, and a downstream charge eliminating member provided in the downstream yarn path guide. The connecting member can include an upstream connecting member that is connected to the upstream charge eliminating member, and a downstream connecting member that is connected to the downstream charge eliminating member. The upstream connecting member can be electrically connected to the downstream connecting member via the second electrically conductive member, and the upstream connecting member or the downstream connecting member can be electrically connected to the substrate via the first electrically conductive member. With such a configuration, because the static electricity is eliminated from the upstream connecting member and the downstream connecting member via the first electrically conductive member, the second electrically conductive member, and the substrate, the effect of the static electricity in the yarn monitoring device can be suppressed.

An electrical resistivity of the connecting member can be higher than or equal to an electrical resistivity of the first electrically conductive member. With such a configuration, the static electricity is guided more effectively from the connecting member to the substrate via the first electrically conductive member. As a result, the effect of the static electricity can be suppressed more effectively in the yarn monitoring device.

The charge eliminating member of the yarn path guide can be made of an electrically conductive material and form a guide surface that guides the yarn. With such a configuration, the static electricity is guided more effectively from the charge eliminating member to the connecting member when the yarn is in direct contact or adjacent to the guide surface of the charge eliminating member that is made of an electrically conductive material. As a result, the effect of the static electricity can be suppressed more effectively in the yarn monitoring device.

The charge eliminating member of the yarn path guide can be made of an electrically conductive resin or an electrically conductive ceramic. With such a configuration, the static electricity is guided more effectively from the charge eliminating member to the connecting member.

The yarn path guide can include a guide main body that includes a guide surface that guides the yarn; and a charge eliminating body that is attached to the guide main body and includes the charge eliminating member. A grooved part that is demarcated by the guide surface can be provided on the guide main body, and the charge eliminating member of the charge eliminating body can be arranged along the grooved part. With such a configuration, because the static electricity is guided more effectively from the guide surface to the connecting member via the charge eliminating body, the static electricity can be eliminated more effectively from the guide surface. As a result, the effect of the static electricity in the yarn monitoring device can be suppressed. Moreover, by attaching the charge eliminating body to the guide main body, the static electricity can be eliminated from the guide surface without changing the configuration of the guide main body.

The electrical resistivity of the charge eliminating member can be lower than or equal to 1.0 × 10⁶ Ω·m. With such a configuration, the static electricity generated in the yarn can be more effectively eliminated by the charge eliminating member.

The yarn path guide can include a tip end edge that guides the traveling yarn; and an inclined surface that is connected to the tip end edge, and extends toward an upstream side and / or downstream side of the tip end edge in the traveling direction of the yarn and inclines away from the traveling space. With such a configuration, for example, because a contact area between the yarn and the yarn path guide is reduced compared to a configuration in which the yarn is guided by a guide surface that is formed as a plane in the traveling direction of the yarn, generation of the static electricity in the yarn and yarn path guide can be suppressed.

### EFFECTS OF THE INVENTION

According to one embodiment of the present invention, it is possible to remove static electricity from the yarn monitoring device. As a result, it is possible to suppress an effect of static electricity on the yarn monitoring device.

In the above explanation, the meaning of "a plurality of" also includes "a predetermined number of".

Although the invention has been explained with respect to specific embodiments for a complete and clear disclosure, the appended claims are not to be thus limited but are to be construed as embodying all modifications and alternative constructions that may occur to one skilled in the art that fall within the scope of the claims.

## Claims

1. A yarn monitoring device (10, 110) that monitors a state of a yarn (Y) that travels in a traveling direction through a traveling space (R1, R2, R3) comprising:
a housing (5); and
a yarn monitoring unit (6, 106) arranged inside the housing (5), wherein
the yarn monitoring unit (6, 106) includes
a monitoring section (63, 64, 163) that monitors a state of the yarn (Y) that travels through the traveling space (R1, R2, R3);
a yarn path guide (65, 165, 66, 166, 90) that guides the traveling yarn (Y); and
a holder (61, 62, 161) that houses the monitoring section (63, 64, 163) and the yarn path guide (65, 165, 66, 166), wherein
the yarn path guide (65, 165, 66, 166) is arranged adjacent to the traveling space (R1, R2, R3),
**characterized in that** the yarn path guide (65, 165, 66, 166) includes a charge eliminating member (65a, 165a, 66a, 166a, 92a) that eliminates an electric charge generated by the traveling of the yarn (y),
the housing (5) includes a connecting member (5, 51, 52, 151, 152) that is electrically connected to the charge eliminating member (65a, 165a, 66a, 166a, 92a), and
an electrical resistivity of the charge eliminating member (65a, 165a, 66a, 166a, 92a) is higher than or equal to an electrical resistivity of the connecting member (5, 51, 52, 151, 152)
wherein the connecting member (5, 51, 52, 151, 152) is electrically connected to a ground potential,
and wherein the yarn monitoring device (10, 110) further comprises
a first electrically conductive member (8, 107a, 153b, 153d) that is arranged in the housing (5); and
a substrate (7, 107) that sends information acquired by the monitoring section (63, 64, 163) to the outside of the yarn monitoring device (10, 110), and is electrically connected to a ground potential, wherein
the connecting member (5, 51, 52, 151, 152) is electrically connected to the substrate (7, 107) via the first electrically conductive member (8, 107a, 153b, 153d).

2. The yarn monitoring device (10, 110) as claimed in Claim 1, wherein
the yarn path guide (65, 165) is an upstream yarn path guide (65, 165) that is provided on an upstream side in the traveling direction of the yarn (Y) with respect to the holder (61, 161), and
the charge eliminating member (65a, 165a) is an upstream charge eliminating member (65a, 165a) that is provided in the upstream yarn path guide (65, 165).

3. The yarn monitoring device (10, 110) as claimed in Claim 1, wherein
the yarn path guide (66, 166) is a downstream yarn path guide (66, 166) that is provided on a downstream side in the traveling direction of the yarn (Y) with respect to the holder (62, 161), and
the charge eliminating member (66a, 166a) is a downstream charge eliminating member (66a, 166a) that is provided in the downstream yarn path guide (66, 166).

4. The yarn monitoring device (10, 110) as claimed in Claim 1, wherein
the yarn path guide (65, 165, 66, 166) includes an upstream yarn path guide (65, 165) that is provided on an upstream side in the traveling direction of the yarn (Y) with respect to the holder (61, 161) and a downstream yarn path guide (66, 166) that is provided on a downstream side in the traveling direction of the yarn (Y) with respect to the holder (62, 161), and
the charge eliminating member (65a, 165a, 66a, 166a) includes an upstream charge eliminating member (65a, 165a) provided in the upstream yarn path guide (65, 165), and a downstream charge eliminating member (66a, 166a) provided in the downstream yarn path guide (66, 166).

5. The yarn monitoring device (10) as claimed in Claim 1, wherein
the yarn path guide (65, 66) includes an upstream yarn path guide (65) that is provided on an upstream side in the traveling direction of the yarn (Y) with respect to the holder (61) and a downstream yarn path guide (66) that is provided on a downstream side in the traveling direction of the yarn (Y) with respect to the holder (62),
the charge eliminating member (65a, 66a) includes an upstream charge eliminating member (65a) provided in the upstream yarn path guide (65), and a downstream charge eliminating member (66a) provided in the downstream yarn path guide (66),
the connecting member (51, 52) includes an upstream connecting member (51) that is connected to the upstream charge eliminating member (65a), and a downstream connecting member (52) that is connected to the downstream charge eliminating member (66a),
the upstream connecting member (51) contacts the downstream connecting member (52), and is electrically connected to the downstream connecting member (52), and
the upstream connecting member (51) or the downstream connecting member (52) is electrically connected to the substrate (7) via the first electrically conductive member (8).

6. The yarn monitoring device (110) as claimed in Claim 1, comprising
a second electrically conductive member (154a) that are arranged in the housing (5); wherein
the yarn path guide (165, 166) includes an upstream yarn path guide (165) that is provided on an upstream side in the traveling direction of the yarn (Y) with respect to the holder (161) and a downstream yarn path guide (166) that is provided on a downstream side in the traveling direction of the yarn (Y) with respect to the holder (161),
the charge eliminating member (165a, 166a) includes an upstream charge eliminating member (165a) provided in the upstream yarn path guide (165), and a downstream charge eliminating member (166a) provided in the downstream yarn path guide (166),
the connecting member (151, 152) includes an upstream connecting member (151) that is connected to the upstream charge eliminating member (165a), and a downstream connecting member (152) that is connected to the downstream charge eliminating member (166a),
the upstream connecting member (151) is electrically connected to the downstream connecting member (152) via the second electrically conductive member (154a), and
the upstream connecting member (151) or the downstream connecting member (152) is electrically connected to the substrate (107) via the first electrically conductive member (107a, 153b, 153d).

7. The yarn monitoring device (10, 110) as claimed in one of Claims 1 to 6, wherein an electrical resistivity of the connecting member (5, 51, 52, 151, 152) is higher than or equal to an electrical resistivity of the first electrically conductive member (8, 107a, 153b, 153d).

8. The yarn monitoring device (10, 110) as claimed in one of Claims 1 to 7, wherein the charge eliminating member (65a, 165a, 66a, 166a) of the yarn path guide (65, 165, 66, 166) is made of an electrically conductive material and forms a guide surface (65b, 165b, 66b, 166b) that guides the yarn (Y).

9. The yarn monitoring device (10, 110) as claimed in Claim 8, wherein the charge eliminating member (65a, 165a, 66a, 166a) of the yarn path guide (65, 165, 66, 166) is made of an electrically conductive resin or an electrically conductive ceramic.

10. The yarn monitoring device (10, 110) as claimed in one of Claims 1 to 7, wherein the yarn path guide (90) includes
a guide main body (91) that includes a guide surface (91a) that guides the yarn (Y); and
a charge eliminating body (92) that is attached to the guide main body (91) and includes the charge eliminating member (92a), wherein
a grooved part (91b) that is demarcated by the guide surface (91a) is provided on the guide main body (91), and
the charge eliminating member (92a) of the charge eliminating body (92) is arranged along the grooved part (91b).

11. The yarn monitoring device (10, 110) as claimed in one of Claims 1 to 10, wherein the electrical resistivity of the charge eliminating member (65a, 165a, 66a, 166a) is lower than or equal to 1.0 × 10⁶ Ω·m.

12. The yarn monitoring device (10, 110) as claimed in one of Claims 1 to 11, wherein the yarn path guide (65A, 65B, 65C, 66A, 66B, 66C) includes
a tip end edge (65f, 66f) that guides the traveling yarn (Y); and
an inclined surface (65d, 66d, 65e, 66e) that is connected to the tip end edge (65f, 66f), and extends toward an upstream side and / or downstream side of the tip end edge (65f, 66f) in the traveling direction of the yarn (Y) and inclines away from the traveling space (R1, R2, R3).

## Patentansprüche

1. Garnüberwachungsvorrichtung (10, 110), die einen Zustand eines Fadens (Y) überwacht, der in einer Laufrichtung durch einen Laufraum (R1, R2, R3) läuft, umfassend:
ein Gehäuse (5); und
eine Fadenüberwachungseinheit (6, 106), die innerhalb des Gehäuses (5) angeordnet ist, wobei
die Fadenüberwachungseinheit (6, 106) Folgendes einschließt:
einen Überwachungsabschnitt (63, 64, 163), der einen Zustand des Fadens (Y) überwacht, der durch den Laufraum (R1, R2, R3) läuft;
eine Fadenlaufführung (65, 165, 66, 166, 90), die den laufenden Faden (Y) führt; und
eine Halterung (61, 62, 161), die den Überwachungsabschnitt (63, 64, 163) und die Fadenlaufführung (65, 165, 66, 166) beherbergt, wobei
die Fadenlaufführung (65, 165, 66, 166) neben dem Laufraum (R1, R2, R3) angeordnet ist, **dadurch gekennzeichnet, dass** die Fadenlaufführung (65, 165, 66, 166) ein Ladungsableiterelement (65a, 165a, 66a, 166a, 92a) einschließt, das eine durch den Lauf des Fadens (y) erzeugte elektrische Ladung ableitet,
das Gehäuse (5) ein Verbindungselement (5, 51, 52, 151, 152) einschließt, das mit dem Ladungsableiterelement (65a, 165a, 66a, 166a, 92a) elektrisch verbunden ist, und
ein elektrischer Widerstand des Ladungsableiterelements (65a, 165a, 66a, 166a, 92a) höher als oder gleich einem elektrischen Widerstand des Verbindungselements (5, 51, 52, 151, 152) ist, wobei das Verbindungselement (5, 51, 52, 151, 152) elektrisch mit einem Massepotential verbunden ist,
und wobei die Garnüberwachungsvorrichtung (10, 110) weiter Folgendes umfasst:
ein erstes elektrisch leitendes Element (8, 107a, 153b, 153d), das in dem Gehäuse (5) angeordnet ist; und
ein Substrat (7, 107), das von dem Überwachungsabschnitt (63, 64, 163) erfasste Informationen an die Außenseite der Garnüberwachungsvorrichtung (10, 110) sendet und elektrisch mit einem Massepotential verbunden ist, wobei
das Verbindungselement (5, 51, 52, 151, 152) über das erste elektrisch leitende Element (8, 107a, 153b, 153d) mit dem Substrat (7, 107) elektrisch verbunden ist.

2. Garnüberwachungsvorrichtung (10, 110) nach Anspruch 1, wobei
die Fadenlaufführung (65, 165) eine stromaufwärtige Fadenlaufführung (65, 165) ist, die in Bezug auf die Halterung (61, 161) auf einer stromaufwärtigen Seite der Laufrichtung des Fadens (Y) bereitgestellt ist, und
das Ladungsableiterelement (65a, 165a) ein stromaufwärtiges Ladungsableiterelement (65a, 165a) ist, das in der stromaufwärtigen Fadenlaufführung (65, 165) bereitgestellt ist.

3. Garnüberwachungsvorrichtung (10, 110) nach Anspruch 1 wobei
die Fadenlaufführung (66, 166) eine stromabwärtige Fadenlaufführung (66, 166) ist, die in Bezug auf die Halterung (62, 161) auf einer stromabwärtigen Seite der Laufrichtung des Fadens (Y) bereitgestellt ist, und
das Ladungsableiterelement (66a, 166a) ein stromabwärtiges Ladungsableiterelement (66a, 166a) ist, das in der stromabwärtigen Fadenlaufführung (66, 166) bereitgestellt ist.

4. Garnüberwachungsvorrichtung (10, 110) nach Anspruch 1, wobei die Fadenlaufführung (65, 165, 66, 166) eine stromaufwärtige Fadenlaufführung (65, 165), die auf einer stromaufwärtigen Seite in der Laufrichtung des Fadens (Y) in Bezug auf die Halterung (61, 161) bereitgestellt ist, und eine stromabwärtige Fadenlaufführung (66, 166) einschließt, die auf einer stromabwärtigen Seite in der Laufrichtung des Fadens (Y) in Bezug auf die Halterung (62, 161) bereitgestellt ist, und
das Ladungsableiterelement (65a, 165a, 66a, 166a) ein stromaufwärtiges Ladungsableiterelement (65a, 165a), das in der stromaufwärtigen Fadenlaufführung (65, 165) bereitgestellt ist, und ein stromabwärtiges Ladungsableiterelement (66a, 166a), das in der stromabwärtigen Fadenlaufführung (66, 166) bereitgestellt ist, einschließt.

5. Garnüberwachungsvorrichtung (10) nach Anspruch 1, wobei
die Fadenlaufführung (65, 66) eine stromaufwärtige Fadenlaufführung (65), die in Bezug auf die Halterung (61) auf einer stromaufwärtigen Seite in der Laufrichtung des Fadens (Y) bereitgestellt ist, und eine stromabwärtige Fadenlaufführung (66), die in Bezug auf die Halterung (62) auf einer stromabwärtigen Seite in der Laufrichtung des Fadens (Y) bereitgestellt ist, einschließt,
das Ladungsableiterelement (65a, 66a) einen stromaufwärtigen Ladungsableiterelement (65a), der in der stromaufwärtigen Fadenlaufführung (65) bereitgestellt ist, und einen stromabwärtigen Ladungsableiterelement (66a), der in der stromabwärtigen Fadenlaufführung (66) bereitgestellt ist, einschließt,
das Verbindungselement (51, 52) ein stromaufwärtiges Verbindungselement (51), das mit dem stromaufwärtigen Ladungsableiterelement (65a) verbunden ist, und ein stromabwärtiges Verbindungselement (52), das mit dem stromabwärtigen Element (66a) verbunden ist, einschließt,
das stromaufwärtige Verbindungselement (51) das stromabwärtige Verbindungselement (52) kontaktiert und elektrisch mit dem stromabwärtigen Verbindungselement (52) verbunden ist und
das stromaufwärtige Verbindungselement (51) oder das stromabwärtige Verbindungselement (52) mit dem Substrat (7) über das erste elektrisch leitende Element (8) elektrisch verbunden ist.

6. Garnüberwachungsvorrichtung (110) nach Anspruch 1, umfassend
ein zweites elektrisch leitendes Element (154a), das in dem Gehäuse (5) angeordnet ist; wobei
die Fadenlaufführung (165, 166) eine stromaufwärtige Fadenlaufführung (165), die in Bezug auf die Halterung (161) auf einer stromaufwärtigen Seite in der Laufrichtung des Fadens (Y) bereitgestellt ist, und eine stromabwärtige Fadenlaufführung (166), die in Bezug auf die Halterung (161) auf einer stromabwärtigen Seite in der Laufrichtung des Fadens (Y) bereitgestellt ist, einschließt,
das Ladungsableiterelement (165a, 166a) ein stromaufwärtiges Ladungsableiterelement (165a), das in der stromaufwärtigen Fadenlaufführung (165) bereitgestellt ist, und ein stromabwärtiges Ladungsableiterelement (166a), das in der stromabwärtigen Fadenlaufführung (166) bereitgestellt ist, einschließt,
das Verbindungselement (151, 152) ein stromaufwärtiges Verbindungselement (151), das mit dem stromaufwärtigen Ladungsableiterelement (165a) verbunden ist, und ein stromabwärtiges Verbindungselement (152), das mit dem stromabwärtigen Ladungsableiterelement (166a) verbunden ist, einschließt,
das stromaufwärtige Verbindungselement (151) mit dem stromabwärtigen Verbindungselement (152) über das zweite elektrisch leitende Element (154a) elektrisch verbunden ist, und
das stromaufwärtige Verbindungselement (151) oder das stromabwärtige Verbindungselement (152) mit dem Substrat (107) über das erste elektrisch leitende Element (107a, 153b, 153d) elektrisch verbunden ist.

7. Garnüberwachungsvorrichtung (10, 110) nach einem der Ansprüche 1 bis 6, wobei der elektrische Widerstand des Verbindungselements (5, 51, 52, 151, 152) höher als oder gleich dem elektrischen Widerstand des ersten elektrisch leitenden Elements (8, 107a, 153b, 153d) ist.

8. Garnüberwachungsvorrichtung (10, 110) nach einem der Ansprüche 1 bis 7, wobei das Ladungsableiterelement (65a, 165a, 66a, 166a) der Fadenlaufführung (65, 165, 66, 166) aus einem elektrisch leitenden Material hergestellt ist und eine Führungsfläche (65b, 165b, 66b, 166b) bildet, die den Faden (Y) führt.

9. Garnüberwachungsvorrichtung (10, 110) nach Anspruch 8, wobei das Ladungsableiterelement (65a, 165a, 66a, 166a) der Fadenlaufführung (65, 165, 66, 166) aus einem elektrisch leitenden Harz oder einer elektrisch leitenden Keramik hergestellt ist.

10. Garnüberwachungsvorrichtung (10, 110) nach einem der Ansprüche 1 bis 7, wobei die Fadenlaufführung (90) Folgendes einschließt:
einen Führungshauptkörper (91), der eine Führungsfläche (91a) beinhaltet, die den Faden (Y) führt; und
einen Ladungsableiterkörper (92), der an dem Führungshauptkörper (91) angebracht ist und das Ladungsableiterelement (92a) einschließt, wobei
ein mit Rillen versehenes Teil (91b), das durch die Führungsfläche (91a) abgegrenzt ist, auf dem Führungshauptkörper (91) bereitgestellt ist, und
das Ladungsableiterelement (92a) des Ladungsableiterkörpers (92) entlang des mit Rillen versehenen Teils (91b) angeordnet ist.

11. Garnüberwachungsvorrichtung (10, 110) nach einem der Ansprüche 1 bis 10, wobei der elektrische Widerstand des Ladungsableiterelements (65a, 165a, 66a, 166a) niedriger als oder gleich 1,0 × 10⁶ Ω·m ist.

12. Garnüberwachungsvorrichtung (10, 110) nach einem der Ansprüche 1 bis 11, wobei die Fadenlaufführung (65A, 65B, 65C, 66A, 66B, 66C)
eine Spitzenendkante (65f, 66f) einschließt, die den laufenden Faden (Y) führt; und
eine geneigte Fläche (65d, 66d, 65e, 66e), die mit der Spitzenendkante (65f, 66f) verbunden ist und sich in Laufrichtung des Fadens (Y) zu einer stromaufwärtigen Seite und/oder einer stromabwärtigen Seite der Spitzenendkante (65f, 66f) erstreckt und sich vom Laufraum (R1, R2, R3) weg neigt.

## Revendications

1. Dispositif de surveillance de fil (10, 110) qui surveille un état d'un fil (Y) qui se déplace dans une direction de déplacement à travers un espace de déplacement (R1, R2, R3), comprenant :
un boîtier (5) ; et
une unité de surveillance du fil (6, 106) agencée à l'intérieur du boîtier (5), dans lequel
l'unité de surveillance du fil (6, 106) inclut
une section de surveillance (63, 64, 163) qui surveille un état du fil (Y) qui se déplace à travers l'espace de déplacement (R1, R2, R3) ;
un guide de chemin de fil (65, 165, 66, 166, 90) qui guide le fil en déplacement (Y) ; et
un support (61, 62, 161) qui abrite la section de surveillance (63, 64, 163) et le guide de chemin de fil (65, 165, 66, 166), dans lequel
le guide de chemin de fil (65, 165, 66, 166) est agencé de manière adjacente à l'espace de déplacement (R1, R2, R3),
**caractérisé en ce que** le guide de chemin de fil (65, 165, 66, 166) inclut un élément d'élimination de charge (65a, 165a, 66a, 166a, 92a) qui élimine une charge électrique générée par le déplacement du fil (y),
le boîtier (5) inclut un élément de connexion (5, 51, 52, 151, 152) qui est connecté électriquement à l'élément d'élimination de charge (65a, 165a, 66a, 166a, 92a), et
une résistivité électrique de l'élément d'élimination de charge (65a, 165a, 66a, 166a, 92a) est supérieure ou égale à une résistivité électrique de l'élément de connexion (5, 51, 52, 151, 152)
dans lequel l'élément de connexion (5, 51, 52, 151, 152) est connecté électriquement à un potentiel de terre,
et dans lequel le dispositif de surveillance de fil (10, 110) comprend en outre
un premier élément électroconducteur (8, 107a, 153b, 153d) qui est agencé dans le boîtier (5) ; et
un substrat (7, 107) qui envoie des informations acquises par la section de surveillance (63, 64, 163) à l'extérieur du dispositif de surveillance de fil (10, 110), et qui est connecté électriquement à un potentiel de terre, dans lequel
l'élément de connexion (5, 51, 52, 151, 152) est connecté électriquement au substrat (7, 107) par l'intermédiaire du premier élément électroconducteur (8, 107a, 153b, 153d).

2. Dispositif de surveillance de fil (10, 110) selon la revendication 1, dans lequel
le guide de chemin de fil (65, 165) est un guide de chemin de fil amont (65, 165) qui est prévu sur un côté amont dans la direction de déplacement du fil (Y) par rapport au support (61, 161), et
l'élément d'élimination de charge (65a, 165a) est un élément d'élimination de charge amont (65a, 165a) qui est prévu dans le guide de chemin de fil amont (65, 165).

3. Dispositif de surveillance de fil (10, 110) selon la revendication 1, dans lequel
le guide de chemin de fil (66, 166) est un guide de chemin de fil aval (66, 166) qui est prévu sur un côté aval dans la direction de déplacement du fil (Y) par rapport au support (62, 161), et
l'élément d'élimination de charge (66a, 166a) est un élément d'élimination de charge aval (66a, 166a) qui est prévu dans le guide de chemin de fil aval (66, 166).

4. Dispositif de surveillance de fil (10, 110) selon la revendication 1, dans lequel le guide de chemin de fil (65, 165, 66, 166) inclut un guide de chemin de fil amont (65, 165) qui est prévu sur un côté amont dans la direction de déplacement du fil (Y) par rapport au support (61, 161) et un guide de chemin de fil aval (66, 166) qui est prévu sur un côté aval dans la direction de déplacement du fil (Y) par rapport au support (62, 161), et
l'élément d'élimination de charge (65a, 165a, 66a, 166a) inclut un élément d'élimination de charge amont (65a, 165a) prévu dans le guide de chemin de fil amont (65, 165), et un élément d'élimination de charge aval (66a, 166a) prévu dans le guide de chemin de fil aval (66, 166).

5. Dispositif de surveillance de fil (10) selon la revendication 1, dans lequel
le guide de chemin de fil (65, 66) inclut un guide de chemin de fil amont (65) qui est prévu sur un côté amont dans la direction de déplacement du fil (Y) par rapport au support (61) et un guide de chemin de fil aval (66) qui est prévu sur un côté aval dans la direction de déplacement du fil (Y) par rapport au support (62),
l'élément d'élimination de charge (65a, 66a) inclut un élément d'élimination de charge amont (65a) prévu dans le guide de chemin de fil amont (65), et un élément d'élimination de charge aval (66a) prévu dans le guide de chemin de fil aval (66),
l'élément de connexion (51, 52) inclut un élément de connexion amont (51) qui est connecté à l'élément d'élimination de charge amont (65a), et un élément de connexion aval (52) qui est connecté à l'élément d'élimination de charge aval (66a),
l'élément de connexion amont (51) entre en contact avec l'élément de connexion aval (52), et est connecté électriquement à l'élément de connexion aval (52), et
l'élément de connexion amont (51) ou l'élément de connexion aval (52) est connecté électriquement au substrat (7) par l'intermédiaire du premier élément électroconducteur (8).

6. Dispositif de surveillance de fil (110) selon la revendication 1, comprenant
un second élément électroconducteur (154a) qui est agencé dans le boîtier (5) ; dans lequel
le guide de chemin de fil (165, 166) inclut un guide de chemin de fil amont (165) qui est prévu sur un côté amont dans la direction de déplacement du fil (Y) par rapport au support (161) et un guide de chemin de fil aval (166) qui est prévu sur un côté aval dans la direction de déplacement du fil (Y) par rapport au support (161),
l'élément d'élimination de charge (165a, 166a) inclut un élément d'élimination de charge amont (165a) prévu dans le guide de chemin de fil amont (165), et un élément d'élimination de charge aval (166a) prévu dans le guide de chemin de fil aval (166),
l'élément de connexion (151, 152) inclut un élément de connexion amont (151) qui est connecté à l'élément d'élimination de charge amont (165a), et un élément de connexion aval (152) qui est connecté à l'élément d'élimination de charge aval (166a),
l'élément de connexion amont (151) est connecté électriquement à l'élément de connexion aval (152) par l'intermédiaire du second élément électroconducteur (154a), et
l'élément de connexion amont (151) ou l'élément de connexion aval (152) est connecté électriquement au substrat (107) par l'intermédiaire du premier élément électroconducteur (107a, 153b, 153d).

7. Dispositif de surveillance de fil (10, 110) selon l'une des revendications 1 à 6, dans lequel une résistivité électrique de l'élément de connexion (5, 51, 52, 151, 152) est supérieure ou égale à une résistivité électrique du premier élément électroconducteur (8, 107a, 153b, 153d).

8. Dispositif de surveillance de fil (10, 110) selon l'une des revendications 1 à 7, dans lequel l'élément d'élimination de charge (65a, 165a, 66a, 166a) du guide de chemin de fil (65, 165, 66, 166) est constitué d'un matériau électroconducteur et forme une surface de guidage (65b, 165b, 66b, 166b) qui guide le fil (Y).

9. Dispositif de surveillance de fil (10, 110) selon la revendication 8, dans lequel l'élément d'élimination de charge (65a, 165a, 66a, 166a) du guide de chemin de fil (65, 165, 66, 166) est constitué d'une résine électroconductrice ou d'une céramique électroconductrice.

10. Dispositif de surveillance de fil (10, 110) selon l'une des revendications 1 à 7, dans lequel le guide de chemin de fil (90) inclut
un corps principal de guide (91) qui inclut une surface de guidage (91a) qui guide le fil (Y) ; et
un corps d'élimination de charge (92) qui est fixé au corps principal de guide (91) et inclut l'élément d'élimination de charge (92a), dans lequel
une partie rainurée (91b) qui est délimitée par la surface de guidage (91a) est prévue sur le corps principal de guide (91), et
l'élément d'élimination de charge (92a) du corps d'élimination de charge (92) est disposé le long de la partie rainurée (91b).

11. Dispositif de surveillance de fil (10, 110) selon l'une des revendications 1 à 10, dans lequel la résistivité électrique de l'élément d'élimination de charge (65a, 165a, 66a, 166a) est inférieure ou égale à 1,0 × 10⁶ Ω·m.

12. Dispositif de surveillance de fil (10, 110) selon l'une des revendications 1 à 11, dans lequel le guide de chemin de fil (65A, 65B, 65C, 66A, 66B, 66C) inclut
un bord d'extrémité de pointe (65f, 66f) qui guide le fil en déplacement (Y) ; et
une surface inclinée (65d, 66d, 65e, 66e) qui est reliée au bord d'extrémité de pointe (65f, 66f) et s'étend vers un côté amont et/ou un côté aval du bord d'extrémité de pointe (65f, 66f) dans la direction de déplacement du fil (Y) et s'incline à l'opposé de l'espace de déplacement (R1, R2, R3).
